(19) Europäisches Patentamt
European Patent Office
Office européen des brevets

(11) Veröffentlichungsnummer: **0 682 021 A1**

(12) **EUROPÄISCHE PATENTANMELDUNG**

(21) Anmeldenummer: **95103231.7**

(22) Anmeldetag: **07.03.95**

(51) Int. Cl.[6]: **C07D 403/04**, C07D 413/14, C07D 417/04, C07D 417/14, C07D 471/04, A61K 31/415, C07D 235/08, C07D 235/18

(30) Priorität: **14.03.94 DE 4408497**

(43) Veröffentlichungstag der Anmeldung: **15.11.95 Patentblatt 95/46**

(84) Benannte Vertragsstaaten: **AT BE CH DE DK ES FR GB GR IE IT LI LU MC NL PT SE**

(71) Anmelder: **Dr. Karl Thomae GmbH**
**Birkendorferstrasse 65**
**D-88397 Biberach (DE)**

(72) Erfinder: **Mihm, Gerhard, Dr. Dipl.-Chem.**
**Scherrichweg 8**
**D-88400 Biberach (DE)**
Erfinder: **Hauel, Norbert, Dr. Dipl.-Chem.**
**Marderweg 12**
**D-88433 Schemmerhofen (DE)**
Erfinder: **Ries, Uwe, Dr. Dipl.-Chem.**
**Dunantstrasse 10**
**D-88400 Biberach (DE)**
Erfinder: **Van Meel, Jacobus Constantinus Antonius, Dr. Phar.**
**Schubertweg 4**
**D-88441 Mittelbiberach (DE)**
Erfinder: **Wienen, Wolfgang, Dr. Dipl.-Biol.**
**Am Schiessberg 28**
**D-88437 Äpfingen (DE)**
Erfinder: **Entzeroth, Michael, Dr. Dipl.-Chem.**
**Sebastian-Sailer-Strasse 44**
**D-88447 Warthausen (DE)**

(54) **Benzimidazole, diese Verbindungen enthaltende Arzneimittel und Verfahren zu ihrer Herstellung.**

(57) Die vorliegende Anmeldung betrifft neue Benzimidazole der allgemeinen Formel

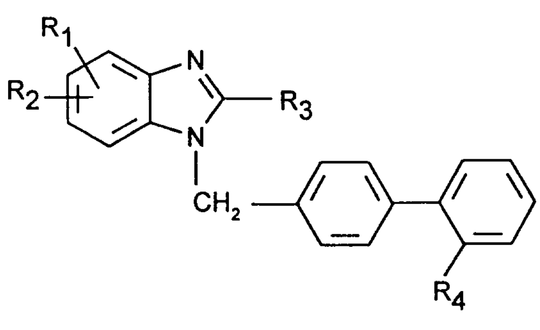

,(I)

in der
$R_1$ bis $R_4$ wie im Anspruch 1 definiert sind, deren Tautomere und deren Salze, insbesondere für die pharmazeutische Verwendung deren physiologisch verträglichen Salze, welche wertvolle pharmakologische Eigenschaften aufweisen, da diese Angiotensin-Antagonisten, insbesondere Angiotensin-II-Antagonisten darstellen, diese Verbindungen enthaltende Arzneimittel, deren Verwendung und Verfahren zu ihrer Herstellung.

EP 0 682 021 A1

Gegenstand der vorliegenden Erfindung sind neue Benzimidazole der allgemeinen Formel

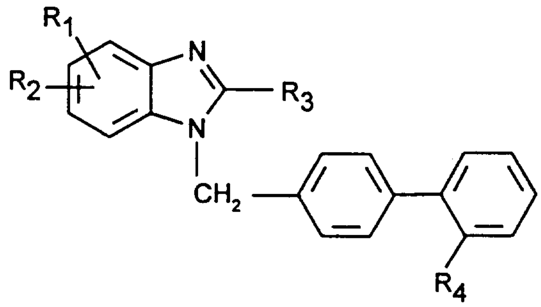

,(I)

deren Tautomere und deren Salze, insbesondere für die pharmazeutische Verwendung deren physiologisch verträglichen Salze, welche wertvolle pharmakologische Eigenschaften aufweisen, da diese Angiotensin-Antagonisten, insbesondere Angiotensin-II-Antagonisten darstellen, diese Verbindungen enthaltende Arzneimittel, deren Verwendung und Verfahren zu ihrer Herstellung.

In der obigen allgemeinen Formel I bedeutet

$R_1$ ein Fluor-, Chlor- oder Bromatom, eine Alkylgruppe mit 1 bis 3 Kohlenstoffatomen oder eine Trifluormethylgruppe,

$R_2$ eine über ein Kohlenstoffatom gebundene 5-gliedrige Heteroarylgruppe, die eine Iminogruppe, ein Sauerstoff- oder Schwefelatom oder eine Iminogruppe und ein Sauerstoff-, Schwefel- oder Stickstoffatom enthält, und an die über zwei benachbarte Kohlenstoffatome eine n-Propylen-, n-Butylen- oder 1,3-Buta-Kohlenstoffatome eine n-Propylen-, n-Butylen- oder 1,3-Butadienylenbrücke oder über eine Iminogruppe und ein benachbartes Kohlenstoffatom eine n-Butylen- oder 1,3-Butadienylenbrücke angefügt sein kann, wobei

das Kohlenstoffgerüst der vorstehend erwähnten bicyclischen Ringe durch ein Fluor-, Chlor- oder Bromatom, durch eine Trifluormethyl-, Alkyl-, Alkoxy-, Cyano-, Aminocarbonyl-, Carboxy-, Nitro-, Amino-, Alkylamino- oder Dialkylaminogruppe mit jeweils 1 bis 3 Kohlenstoffatomen im Alkyl- und Alkoxyteil mono- oder disubstituiert sein kann, wobei die Substituenten gleich oder verschieden sein können, und zusätzlich eine HN-Gruppe der vorstehend erwähnten heteroaromatischen Ringe durch eine Alkylgruppe mit 1 bis 6 Kohlenstoffatomen, durch eine Phenylalkylgruppe mit 1 bis 3 Kohlenstoffatomen im Alkylteil oder durch eine Cycloalkylgruppe mit 3 bis 6 Kohlenstoffatomen substituiert sein kann,

eine durch eine Alkylgruppe mit 1 bis 3 Kohlenstoffatomen substituierte Imidazol-4-yl-Gruppe, in der der Alkylteil in 2- oder 3-Stellung durch eine Dimethylamino-, Diethylamino-, Pyrrolidino-, Piperidino-, Hexamethylenimino- oder Morpholinogruppe substituiert sein kann,

eine Pyrrolidino-, Piperidino- oder Hexamethyleniminogruppe, in denen jeweils eine Methylengruppe durch eine Carbonyl- oder Sulfonylgruppe ersetzt ist,

eine gegebenenfalls durch eine Alkylgruppe mit 1 bis 3 Kohlenstoffatomen oder durch eine Phenylgruppe substituierte Maleinsäureimidogruppe oder

eine gegebenenfalls durch eine Alkylgruppe mit 1 bis 3 Kohlenstoffatomen substituierte Imidazolidin-2,4-dion-3-yl-Gruppe,

$R_3$ eine Alkylgruppe mit 1 bis 4 Kohlenstoffatomen, eine Cycloalkylgruppe mit 3 bis 5 Kohlenstoffatomen, eine Alkoxy- oder Alkylthiogruppe mit jeweils 1 bis 3 Kohlenstoffatomen im Alkylteil und

$R_4$ eine gegebenenfalls durch eine Alkoxycarbonylgruppe mit insgesamt 2 bis 6 Kohlenstoffatomen, durch eine Trifluoracetyl- oder Trifluormethylsulfonylgruppe substituierte Aminogruppe,

eine durch eine Hydroxy-, Amino-, Alkylcarbonylamino-, Alkoxycarbonylamino, Alkylaminocarbonylamino-, Dialkylaminocarbonylamino-, Cycloalkylcarbonylamino-, Cycloalkylaminocarbonylamino-, Phenylcarbonylamino-, Phenylaminocarbonylamino-, Phenylalkylcarbonylamino- oder Phenylalkylaminocarbonylaminogruppe substituierte Sulfonylgruppe, in denen der Alkylteil jeweils 1 bis 3 Kohlenstoffatome, der Cycloalkylteil jeweils 5 bis 7 Kohlenstoffatome enthalten und der Phenylkern jeweils durch ein Fluor-, Chlor- oder Bromatom oder durch eine Methoxygruppe substituiert sein kann,

eine Hydroxycarbamidoyl-, Thiazolidin-2,4-dion-5-methyliden- oder 2,5-Dihydro-5-oxo-oxadiazol-3-yl-Gruppe.

Für die bei der Definition der Reste $R_1$ bis $R_4$ in den Ansprüchen 1 bis 5 erwähnten Bedeutungen kommt beispielsweise für

$R_1$ die Bedeutung des Fluor-, Chlor- oder Bromatoms, der Methyl-, Ethyl-, n-Propyl-, Isopropyl- oder

Trifluormethylgruppe,

$R_2$ die der 1-Methyl-imidazol-4-yl-, 1-Ethyl-imidazol-4-yl-, 1-n-Propyl-imidazol-4-yl-, 1-Isopropyl-imidazol-4-yl-, 1-(2-Dimethylamino-ethyl)-imidazol-4-yl-, 1-(3-Dimethylamino-propyl)-imidazol-4-yl-, 1-(2-Diethylamino-ethyl)-imidazol-4-yl-, 1-(3-Diethylamino-propyl)-imidazol-4-yl-, 1-(2-Pyrrolidino-ethyl)-imidazol-4-yl-, 1-(3-Pyrrolidino-propyl)-imidazol-4-yl-, 1-(2-Piperidino-ethyl)-imidazol-4-yl-, 1-(3-Piperidino-propyl)-imidazol-4-yl-, 1-(2-Hexamethylenimino-ethyl)-imidazol-4-yl-, 1-(3-Hexamethylenimino-propyl)-imidazol-4-yl-, 1-(2-Morpholino-ethyl)-imidazol-4-yl-, 1-(3-Morpholino-propyl)-imidazol-4-yl-, 2-Oxo-pyrrolidino-, 2-Oxo-piperidino-, 2-Oxo-hexamethylenimino-, Propansultam-1-yl-, Butansultam-1-yl-, Pentansultam-1-yl-, Maleinsäureimido-, 2-Methyl-maleinsäureimido-, 2-Phenyl-maleinsäureimido-, 4,5-Trimethylen-imidazol-2-yl-, 4,5-Trimethylen-1-methyl-imidazol-2-yl-, 4,5-Trimethylen-1-n-butyl-imidazol-2-yl-, 4,5-Trimethylen-1-n-hexyl-imidazol-2-yl-, 4,5-Trimethylen-1-cyclopropyl-imidazol-2-yl-, 4,5-Trimethylen-1-cyclohexyl-imidazol-2-yl-, Benzimidazol-2-yl-, 1-Methylbenzimidazol-2-yl-, 1-Ethylbenzimidazol-2-yl-, 1-n-Propylbenzimidazol-2-yl-, 1-Isopropylbenzimidazol-2-yl-, 1-n-Butylbenzimidazol-2-yl-, 1-Isobutylbenzimidazol-2-yl-, 1-n-Pentylbenzimidazol-2-yl-, 1-n-Hexylbenzimidazol-2-yl-, 1-Cyclopropylbenzimidazol-2-yl-, 1-Cyclobutylbenzimidazol-2-yl-, 1-Cyclopentylbenzimidazol-2-yl-, 1-Cyclohexylbenzimidazol-2-yl-, 5-Nitrobenzimidazol-2-yl-, 5-Amino-benzimidazol-2-yl-, 5-Acetamidobenzimidazol-2-yl-, 5-Methyl-benzimidazol-2-yl-, 5-Methoxybenzimidazol-2-yl-, 5-Ethoxy-benzimidazol-2-yl-, 1-Methyl-5-methoxybenzimidazol-2-yl-,1,5-Dimethyl-benzimidazol-2-yl-, 1,6-Dimethyl-benzimidazol-2-yl-,1,4-Dimethyl-benzimidazol-2-yl-, 5,6-Dimethyl-benzimidazol-2-yl-,1,5,6-Trimethyl-benzimidazol-2-yl-, 5-Chlor-benzimidazol-2-yl-, 5-Chlor-1-methyl-benzimidazol-2-yl-,6-Chlor-1-methyl-benzimidazol-2-yl-, 5,6-Dichlor-1-methyl-benzimidazol-2-yl-, 5-Dimethylamino-benzimidazol-2-yl-, 5-Dimethylamino-1-ethyl-benzimidazol-2-yl-, 5,6-Dimethoxy-1-methyl-benzimidazol-2-yl-, 5,6-Dimethoxy-1-ethyl-benzimidazol-2-yl-, 5-Fluor-1-methyl-benzimidazol-2-yl-, 6-Fluor-1-methyl-benzimidazol-2-yl-, 5-Trifluormethyl-benzimidazol-2-yl-, 5-Trifluormethyl-1-methyl-benzimidazol-2-yl-, 4-Cyano-1-methyl-benzimidazol-2-yl-, 5-Carboxy-1-methyl-benzimidazol-2-yl-, 5-Aminocarbonyl-benzimidazol-2-yl-, 5-Aminocarbonyl-1-methyl-benzimidazol-2-yl-, 4,5,6,7-Tetrahydro-benzimidazol-2-yl-, 4,5,6,7-Tetrahydro-1-methyl-benzimidazol-2-yl-, 4,5,6,7-Tetrahydro-1-ethyl-benzimidazol-2-yl-, 4,5,6,7-Tetrahydro-1-n-butyl-benzimidazol-2-yl-, 4,5,6,7-Tetrahydro-1-n-hexyl-benzimidazol-2-yl-, 4,5,6,7-Tetrahydro-1-cyclopropyl-benzimidazol-2-yl-, 4,5,6,7-Tetrahydro-1-cyclohexyl-benzimidazol-2-yl-, Imidazo[1,2-a]pyridin-2-yl-, 5-Methyl-imidazo[1,2-a]pyridin-2-yl-, 6-Methyl-imidazo[1,2-a]pyridin-2-yl-, 7-Methyl-imidazo[1,2-a]pyridin-2-yl-, 8-Methyl-imidazo[1,2-a]pyridin-2-yl-, 5,7-Dimethyl-imidazo[1,2-a]pyridin-2-yl-, 5,6,7,8-Tetrahydro-imidazo[1,2-a]pyridin-2-yl-, Imidazo[1,2-a]pyrimidin-2-yl-, Imidazo[4,5-b]pyridin-2-yl-, 1-Methyl-imidazo[4,5-b]pyridin-2-yl-, 1-n-Hexyl-imidazo[4,5-b]pyridin-2-yl-, 1-Cyclopropyl-imidazo[4,5-b]-pyridin-2-yl-, 1-Cyclohexyl-imidazo[4,5-b]pyridin-2-yl-, 6-Methyl-imidazo[4,5-b]pyridin-2-yl-, 1,6-Dimethyl-imidazo[4,5-b]pyridin-2-yl-, Imidazo[4,5-c]pyridin-2-yl-, 1-Methyl-imidazo-[4,5-c]pyridin-2-yl-, 1-n-Hexyl-imidazo[4,5-c]pyridin-2-yl-, 1-Cyclopropyl-imidazo[4,5-c]pyridin-2-yl-, 1-Cyclohexyl-imidazo[4,5-c]pyridin-2-yl-, Imidazo[2,1-b]thiazol-6-yl-, Imidazo[1,2-c]pyrimidin-2-yl-, Imidazo[1,2-a]pyrazin-2-yl-, Imidazo[1,2-b]-pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl-, Imidazolidin-2,4-dion-3-yl- oder 5-Methyl-imidazolidin-2,4-dion-3-yl-Gruppe,

für $R_3$ die der Methyl-, Ethyl-, n-Propyl-, Isopropyl-, n-Butyl, 1-Methyl-n-propyl-, 2-Methyl-n-propyl-, tert.Butyl-, Cyclopropyl-, Cyclobutyl-, Cyclopentyl-, Methoxy-, Ethoxy-, n-Propoxy-, Isopropoxy-, Methylthio-, Ethylthio-, n-Propylthio- oder Isopropylthiogruppe und

für $R_4$ die der Formyl-, Hydroxymethyl-, Amino-, Methoxycarbonylamino-, Ethoxycarbonylamino-, Propoxycarbonylamino-, tert.Butoxycarbonylamino-, Trifluoracetylamino-, Trifluormethylsulfonylamino-, Sulfo-, Sulfamoyl-, Methoxycarbonylaminosulfonyl-, Ethoxycarbonylaminosulfonyl-, Propoxycarbonylaminosulfonyl-, tert.Butoxycarbonylaminosulfonyl-, Acetylaminosulfonyl-, Propionylaminosulfonyl-, Butanoylaminosulfonyl-, Methylaminocarbonylaminosulfonyl-, Ethylaminocarbonylaminosulfonyl-, n-Propylaminocarbonylaminosulfonyl-, Isopropylaminocarbonylaminosulfonyl-, Methylaminocarbonylaminosulfonyl-, N,N-Dimethylaminocarbonylaminosulfonyl-, N,N-Diethylaminocarbonylaminosulfonyl-, Cyclohexylcarbonylaminosulfonyl-, Cycloheptylcarbonylaminosulfonyl-, Cyclopentylaminocarbonylaminosulfonyl-, Cyclohexylaminocarbonylaminosulfonyl-, Cycloheptylaminocarbonylaminosulfonyl-, Phenylcarbonylaminosulfonyl-, 4-Fluor-phenylcarbonylaminosulfonyl-, 4-Chlor-phenylcarbonylaminosulfonyl-, 4-Brom-phenylcarbonylaminosulfonyl-,4-Methoxy-phenylcarbonylaminosulfonyl-, Phenylaminocarbonylaminosulfonyl-, 4-Fluor-phenylaminocarbonylaminosulfonyl-,4-Chlor-phenylaminocarbonylaminosulfonyl-, 4-Brom-phenylaminocarbonylaminosulfonyl-, 4-Methoxy-phenylaminocarbonylaminosulfonyl-, Benzylaminocarbonylaminosulfonyl-, 4-Fluor-benzylaminocarbonylaminosulfonyl-, 4-Chlor-benzylaminocarbonylaminosulfonyl-, 4-Brom-benzylaminocarbonylaminosulfonyl-, 4-Methoxy-benzylaminocarbonylaminosulfonyl-, 2-Phenylethylaminocarbonylaminosulfonyl-, 2-(4-Fluor-phenyl)-ethylaminocarbonylaminosulfonyl-, 2-(4-Chlor-phenyl)ethylaminocarbonylaminosulfonyl-, 2-(4-Brom-phenyl)-ethylaminocarbonylaminosulfonyl-, 2-(4-Methoxy-phenyl)ethylaminocarbonylaminosulfonyl-, 3-Phenylpropylaminocarbonylaminosulfonyl-, 3-(4-Fluor-phenyl)-propylaminocarbonylaminosulfonyl-, 3-(4-Chlor-phenyl)-

propylaminocarbonylaminosulfonyl-, 3-(4-Brom-phenyl)propylaminocarbonylaminosulfonyl-, 3-(4-Methoxyphenyl)propylaminocarbonylaminosulfonyl-, Hydroxycarbamidoyl-, 1,3-Thiazolidin-2,4-dion-5-methyliden- oder 1,2,4-Oxadiazol-5-on-3-yl-Gruppe in Betracht.

Bevorzugte Verbindungen der obigen allgemeinen Formel I sind diejenigen, in denen
$R_1$ eine Methylgruppe,
$R_2$ eine gegebenenfalls in 1-Stellung durch eine Methylgruppe substituierte Benzimidazol-2-yl-Gruppe, eine gegebenenfalls in 1-Stellung durch eine Alkylgruppe mit 1 bis 3 Kohlenstoffatomen subsituierte Imidazol-4-yl-Gruppe, in der der Alkylsubstituent in 2- oder 3-Stellung durch eine Morpholinogruppe substituiert sein kann, eine 5,6,7,8-Tetrahydro-imidazo[1,2-a]pyridin-2-yl-, Propansultam-1-yl- oder Butansultam-1-yl-Gruppe,
$R_3$ eine geradkettige Alkylgruppe mit 2 bis 4 Kohlenstoffatomen und
$R_4$ eine Aminogruppe, eine durch eine Hydroxy-, Amino-, Dimethylaminocarbonylamino-, Phenylcarbonylamino-, Cycloalkylaminocarbonylamino- oder Benzylaminocarbonylaminogruppe substituierte Sulfonylgruppe, in denen der Cycloalkylteil jeweils 5 oder 6 Kohlenstoffatome enthalten und der Phenylteil jeweils durch eine Methoxygruppe substituiert sein kann, eine Trifluoracetylamino-, tert.Butoxycarbonylamino-, Trifluormethylsulfonylamino-, 1,3-Thiazolidin-2,4-dion-5-methyliden- oder 1,2,4-Oxadiazol-5-on-3-yl-Gruppe bedeuten,
deren Tautomere und deren Salze.

Besonders bevorzugte Verbindungen der obigen allgemeinen Formel I sind diejenigen, in denen
$R_1$ in 4-Stellung eine Methylgruppe,
$R_2$ in 6-Stellung eine gegebenenfalls in 1-Stellung durch eine Methylgruppe substituierte Benzimidazol-2-yl-Gruppe, eine in 1-Stellung durch eine Alkylgruppe mit 1 bis 3 Kohlenstoffatomen subsituierte Imidazol-4-yl-Gruppe, in der der Alkylsubstituent in 2- oder 3-Stellung durch eine Morpholinogruppe substituiert sein kann, eine 5,6,7,8-Tetrahydro-imidazo[1,2-a]pyridin-2-yl-, Propansultam-1-yl- oder Butansultam-1-yl-Gruppe,
$R_3$ eine geradkettige Alkylgruppe mit 2 bis 4 Kohlenstoffatomen und
$R_4$ eine durch eine Hydroxy-, Amino-, Dimethylaminocarbonylamino-, Phenylcarbonylamino-, Cycloalkylaminocarbonylamino- oder Benzylaminocarbonylaminogruppe substituierte Sulfonylgruppe, in denen der Cycloalkylteil jeweils 5 oder 6 Kohlenstoffatome enthalten und der Phenylteil jeweils durch eine Methoxygruppe substituiert sein kann, eine Trifluoracetylamino-, Trifluormethylsulfonylamino-, 1,3-Thiazolidin-2,4-dion-5-methyliden- oder 1,2,4-Oxadiazol-5-on-3-yl-Gruppe bedeuten, und
deren Salze.

Ganz besonders bevorzugte Verbindungen der obigen allgemeinen Formel I sind diejenigen, in denen
$R_1$ in 4-Stellung eine Methylgruppe,
$R_2$ in 6-Stellung eine 1-Methyl-benzimidazol-2-yl-, 1-(2-Morpholinoethyl)-imidazol-4-yl-, 5,6,7,8-Tetrahydro-imidazo[1,2-a]pyridin-2-yl- oder Butansultam-1-yl-Gruppe,
$R_3$ eine Ethyl- oder n-Propylgruppe und
$R_4$ eine durch eine Hydroxy-, Dimethylaminocarbonylamino-, Phenylcarbonylamino-, Cycloalkylaminocarbonylamino- oder Benzylaminocarbonylaminogruppe substituierte Sulfonylgruppe, in denen der Cycloalkylteil jeweils 5 oder 6 Kohlenstoffatome enthalten und der Phenylteil jeweils durch eine Methoxygruppe substituiert sein kann, eine Trifluoracetylamino-, Trifluormethylsulfonylamino-, 1,3-Thiazolidin-2,4-dion-5-methyliden- oder 1,2,4-Oxadiazol-5-on-3-yl-Gruppe bedeuten, und
deren Salze.

Als bevorzugte Verbindungen seien beispielsweise folgende genannt:

(a) 4'-[(2-n-Propyl-4-methyl-6-(1-methylbenzimidazol-2-yl)-benzimidazol-1-yl)-methyl]-2-(1,3-thiazolidin-2,4-dion-5-methylidinyl)-biphenyl,

(b) 4'-[(2-n-Propyl-4-methyl-6-(1-methylbenzimidazol-2-yl)-benzimidazol-1-yl)-methyl]-2-sulfo-biphenyl,

(c) 4'-[(2-Ethyl-4-methyl-6-(5,6,7,8-tetrahydro-imidazo[1,2-a]pyridin-2-yl)-benzimidazol-1-yl)-methyl]-2-sulfo-biphenyl,

(d) 4'-[(2-n-Propyl-4-methyl-6-(1-methylbenzimidazol-2-yl)-benzimidazol-1-yl)-methyl]-2-trifluoracetylamino-biphenyl,

(e) 4'-[(2-Ethyl-4-methyl-6-(5,6,7,8-tetrahydro-imidazo[1,2-a]pyridin-2-yl)-benzimidazol-1-yl)-methyl]-2-trifluoracetylaminobiphenyl,

(f) 4'-[(2-n-Propyl-4-methyl-6-(1-methylbenzimidazol-2-yl)-benzimidazol-1-yl)-methyl]-2-(4-methoxy-benzylamino-carbonylaminosulfonyl)-biphenyl,

(g) 4'-[(2-n-Propyl-4-methyl-6-(1-methylbenzimidazol-2-yl)-benzimidazol-1-yl)-methyl]-2-(cyclohexylaminocarbonylaminosulfonyl)-biphenyl,

(h) 4'-[(2-n-Propyl-4-methyl-6-(1-methylbenzimidazol-2-yl)-benzimidazol-1-yl)-methyl]-2-(benzoylaminosulfonyl)-biphenyl,

(i) 4'-[(2-Ethyl-4-methyl-6-(5,6,7,8-tetrahydro-imidazo[1,2-a]pyridin-2-yl)-benzimidazol-1-yl)-methyl]-2-(benzoylaminosulfonyl)-biphenyl,

(j) 4'-[(2-n-Butyl-4-methyl-6-(propansultam-1-yl)-benzimidazol-1-yl)-methyl]-2-(benzoylaminosulfonyl)-biphenyl und

(k) 4'-[(2-Ethyl-4-methyl-6-(5,6,7,8-tetrahydro-imidazo[1,2-a]pyridin-2-yl)-benzimidazol-1-yl)-methyl]-2-(cyclohexylaminocarbonylaminosulfonyl)-biphenyl

sowie deren physiologisch verträgliche Salze.

Erfindungsgemäß erhält man die neuen Verbindungen nach folgenden Verfahren:

a) Umsetzung eines Benzimidazols der allgemeinen Formel

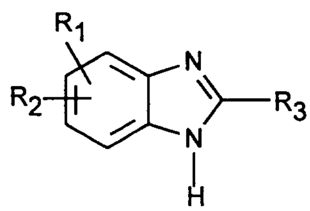

,( II )

in der

$R_1$ bis $R_3$ wie eingangs definiert sind, mit einer Biphenylverbindung der allgemeinen Formel

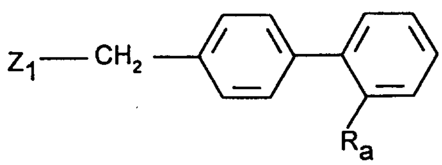

,( III )

in der

$R_a$ die für $R_4$ eingangs erwähnten Bedeutungen besitzt, wobei jedoch ein vorhandenes reaktives Wasserstoffatom durch einen üblichen Schutzrest wie eine Alkoxycarbonylgruppe mit insgesamt 2 bis 6 Kohlenstoffatomen oder eine Benzyloxycarbonylgruppe geschützt ist und

$Z_1$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Benzolsulfonyloxy- oder p-Toluolsulfonyloxygruppe, darstellt, und gegebenenfalls anschließende Abspaltung eines verwendeten Schutzrestes.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid, Dimethylacetamid oder Benzol gegebenenfalls in Gegenwart eines säurebindenden Mittels, wie Natriumcarbonat, Kaliumcarbonat, Natriumhydroxid, Kalium-tert.butylat, Natriumhydrid, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Die gegebenenfalls anschließende Abspaltung eines gegebenenfalls verwendeten Schutzrestes erfolgt vorzugsweise hydrolytisch in Gegenwart einer Säure wie Salzsäure, Schwefelsäure, Phosphorsäure, Trichloressigesäure oder Trifluoressigsäure oder in Gegenwart einer Base wie Natriumhydroxid oder Kaliumhydroxid in einem geeigneten Lösungsmittel wie Wasser, Wasser/Methanol, Ethanol, Ethanol/Wasser, Wasser/Isopropanol oder Wasser/Dioxan oder in Gegenwart eines primären Amins wie Methylamin, Ethylamin oder Propylamin bei Temperaturen zwischen -10 und 120°C, z.B. bei Temperaturen zwischen Raumtemperatur und der Siedetemperatur des Reaktionsgemisches.

Bei der Umsetzung erhält man vorzugsweise ein Gemisch der 1- und 3-Isomeren, aus welchem anschließend durch Kristallisation oder chromatographisch unter Verwendung eines Trägers wie Kieselgel oder Aluminiumoxid, das entsprechende 1-Isomere abgetrennt wird.

b) Zur Herstellung von Verbindungen der allgemeinen Formel I, in der $R_4$ eine Hydroxymethylgruppe darstellt:

Reduktion einer Verbindung der allgemeinen Formel

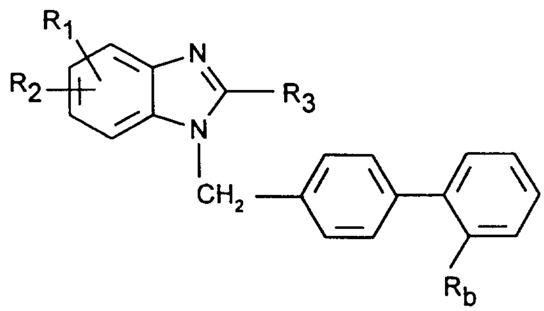

,( IV )

in der
$R_1$ bis $R_3$ wie eingangs definiert sind und
$R_b$ eine veresterte Carboxygruppe, z.B. eine Alkoxycarbonylgruppe mit insgesamt 2 bis 10 Kohlenstoffatomen, eine Phenylalkoxycarbonylgruppe mit 1 bis 4 Kohlenstoffatomen im Alkylteil oder eine Phenyloxycarbonylgruppe, darstellt.

Die Reduktion wird in einem geeigneten Lösungsmittel wie Methanol, Methanol/Wasser, Ethanol, Ether, Tetrahydrofuran, Dioxan oder Eisessig in Gegenwart eines Metallhydrids wie Natriumborhydrid, Lithiumborhydrid oder Lithiumaluminiumhydrid bei Temperaturen zwischen 0 und 50 °C, vorzugsweise jedoch bei Temperaturen zwischen 15 und 25 °C, durchgeführt.

c) Zur Herstellung von Verbindungen der allgemeinen Formel I, in der $R_4$ eine Formylgruppe darstellt:
Oxidation einer Verbindung der allgemeinen Formel

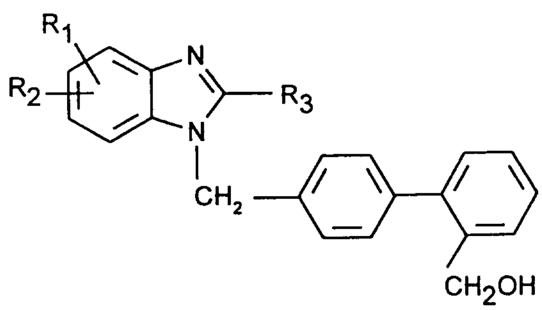

,( V )

in der
$R_1$ bis $R_3$ wie eingangs definiert sind.

Die Oxidation wird vorzugsweise in einem Lösungsmittel oder Lösungsmittelgemisch, z.B. in Wasser, Wasser/Pyridin, Aceton, Eisessig, Methylenchlorid, Eisessig/Acetanhydrid, verdünnter Schwefelsäure oder Trifluoressigsäure, mit einem Oxidationsmittel bei Temperaturen zwischen 0 und 80 °C, vorzugsweise bei Temperaturen zwischen 15 und 25 °C, z.B. mit Braunstein, Kaliumpermanganat oder Rutheniumdioxid bei 20 °C, mit Chromsäure in Eisessig, Schwefelsäure, Pyridin oder in Aceton bei 0 bis 20 °C oder mit Dimethylsulfoxid/Oxalylchlorid bei 0 bis 20 °C, durchgeführt.

d) Zur Herstellung von Verbindungen der allgemeinen Formel I, in der $R_4$ eine Thiazolidin-2,4-dion-5-methylidengruppe darstellt:
Umsetzung einer Verbindung der allgemeinen Formel

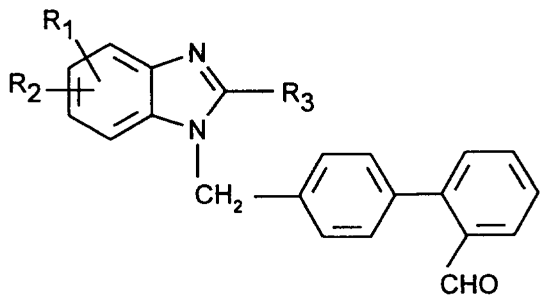

,( VI )

in der
$R_1$ bis $R_3$ wie eingangs definiert sind, mit
Thiazolidin-2,4-dion.

Die Umsetzung wird vorzugsweise in einem Lösungsmittel wie Eisessig, Propionsäure oder Acetanhydrid bei erhöhten Temperaturen, z.B. bei Temperaturen zwischen 80 und 120 °C, vorzugsweise jedoch bei der Siedetemperatur des verwendeten Lösungsmittels durchgeführt.

e) Zur Herstellung von Verbindungen der allgemeinen Formel I, in der $R_4$ eine gegebenenfalls durch eine Alkoxycarbonylgruppe mit insgesamt 2 bis 6 Kohlenstoffatomen substituierte Aminogruppe darstellt:
Umsetzung einer gegebenenfalls im Reaktionsgemisch gebildeten Verbindung der allgemeinen Formel

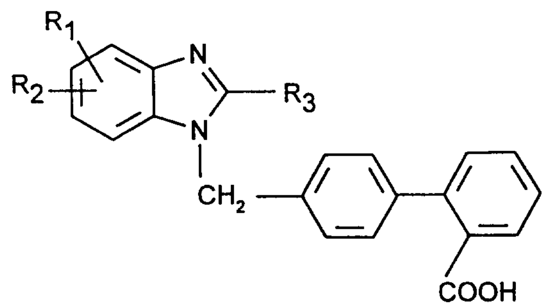

,( VII )

in der
$R_1$ bis $R_3$ wie eingangs definiert sind, mit Natriumazid, anschließende Umsetzung mit Wasser oder einem aliphatischen Alkohol mit 1 bis 5 Kohlenstoffatomen und gegebenenfalls anschließende Abspaltung einer Alkoxycarbonylgruppe mit 2 bis 6 Kohlenstoffatomen.

Die Umsetzung wird vorzugsweise in einem Lösungsmittel wie Chloroform/Wasser mit Natriumazid in Gegenwart von einem Tetraalkylammoniumchlorid wie Tetrabutylammoniumchlorid bei Temperaturen zwischen -5 und 20 °C, vorzugsweise jedoch bei 0 °C, durchgeführt.

Die anschließende Umsetzung mit Wasser oder einem Alkohol erfolgt vorzugsweise in einem wässrigen Lösungsmittel, z.B. in Wasser, Isopropanol/Wasser, Tetrahydrofuran/Wasser oder Dioxan/Wasser oder in Gegenwart eines Alkohols wie Methanol, Ethanol, Propanol, Isopropanol, tert.Butanol oder Pentanol bei Temperaturen zwischen 0 und 100 °C, vorzugsweise bei der Siedetemperatur des Reaktionsgemisches.

Die gegebenenfalls anschließende Abspaltung eines gegebenenfalls verwendeten Schutzrestes erfolgt vorzugsweise hydrolytisch in Gegenwart einer Säure wie Salzsäure, Schwefelsäure, Phosphorsäure, Trichloressigesäure oder Trifluoressigsäure oder in Gegenwart einer Base wie Natriumhydroxid oder Kaliumhydroxid in einem geeigneten Lösungsmittel wie Wasser, Wasser/Methanol, Ethanol, Ethanol/Wasser, Wasser/Isopropanol oder Wasser/Dioxan oder in Gegenwart eines primären Amins wie Methylamin, Ethylamin oder Propylamin bei Temperaturen zwischen -10 und 120 °C, z.B. bei Temperaturen zwischen Raumtemperatur und der Siedetemperatur des Reaktionsgemisches.

f) Zur Herstellung von Verbindungen der allgemeinen Formel I, in der $R_4$ eine Sulfogruppe darstellt:
Umsetzung einer Verbindung der allgemeinen Formel

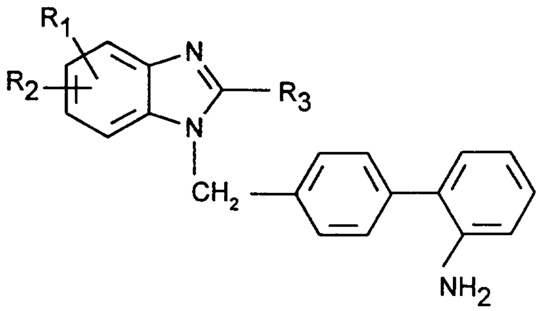

,( VIII )

in der
$R_1$ bis $R_3$ wie eingangs definiert sind, mit einem Nitrit und anschließende Umsetzung mit Schwefeldioxid.

Das Diazoniumsalz wird zweckmäßigerweise in einem Lösungsmittel, z.B. in Wasser/Salzsäure, Wasser/Schwefelsäure, Methanol/Salzsäure, Ethanol/Salzsäure oder Dioxan/Salzsäure, durch Diazotierung einer Verbindung der allgemeinen Formel VIII mit einem Nitrit, z.B. Natriumnitrit oder einem Ester der salpetrigen Säuren, bei niederen Temperaturen, z.B. bei Temperaturen zwischen -10 und 5°C, hergestellt. Die anschließende Umsetzung mit Schwefeldioxid wird zweckmäßigerweise in Gegenwart von Kupfer(II)chlorid in einem Lösungsmittel wie Wasser, Methanol/Wasser oder Wasser/Salzsäure bei niederen Temperaturen, z.B. bei Temperaturen zwischen -10 und 5°C, durchgeführt.

g) Zur Herstellung von Verbindungen der allgemeinen Formel I, in der $R_4$ eine durch eine Alkoxycarbonylgruppe mit insgesamt 2 bis 6 Kohlenstoffatomen, durch eine Trifluoracetyl- oder Trifluormethylsulfonylgruppesubstituierte Aminogruppe darstellt:
Umsetzung einer Verbindung der allgemeinen Formel

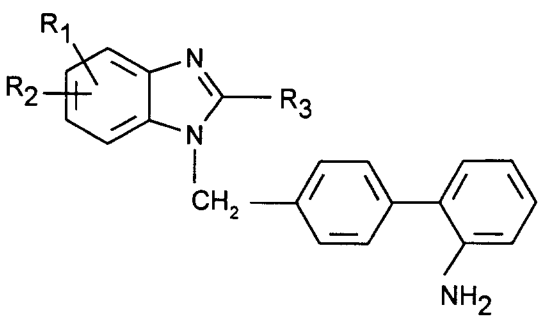

,( VIII )

in der
$R_1$ bis $R_3$ wie eingangs definiert sind, mit einer Verbindung der allgemeinen Formel

$Z_2$-X-$R_c$ , (IX)

in der
$R_c$ eine Alkoxygruppe mit 1 bis 5 Kohlenstoffatomen oder eine Trifluormethylgruppe,
X eine Carbonyl- oder Sulfonylgruppe und
$Z_2$ eine Austrittsgruppe wie ein Halogenatom, z.B. ein Chlor- oder Bromatom, eine Azidogruppe oder eine Acyloxygruppe, z.B. die Acetoxy-, Methoxycarbonyloxy-, Ethoxycarbonyloxy- oder Isobutoxycarbonyloxygruppe oder, falls $R_c$ eine Trifluormethylgruppe bedeutet, auch eine Hydroxygruppe, bedeuten.

Die Umsetzung wird zweckmäßigerweise in einem Lösungsmittel wie Methanol, Methylenchlorid, Chloroform, Tetrachlorkohlenstoff, Ether, Tetrahydrofuran, Dioxan, Benzol, Toluol, Acetonitril, Sulfolan oder Dimethylformamid gegebenenfalls in Gegenwart einer anorganischen oder organischen Base, gegebenenfalls in Gegenwart eines die Säure aktivierenden Mittels, gegebenenfalls in Gegenwart eines wasserentziehenden Mittels oder gegebenenfalls eines die Aminogruppe aktivierenden Mittels bei Temperaturen zwischen -20 und 200°C, vorzugsweise jedoch bei Temperaturen zwischen -10 und 160°C, durchgeführt. Bedeutet $Z_2$ eine Hydroxygruppe, so wird die Acylierung zweckmäßigerweise in einem

Lösungsmittel wie Tetrahydrofuran, Ethylenchlorid, Chloroform, Sulfolan oder Dimethylformamid in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer Base wie Natriumcarbonat, Kaliumcarbonat, Kalium-tert.butylat oder 1-Hydroxy-benztriazol/Triethylamin oder in Gegenwart einer tertiären organischen Base wie 4-Dimethylamino-pyridin, Triethylamin, N-Ethyl-diisopropylamin, N-Methyl-morpholin oder Pyridin, welche gleichzeitig auch als Lösungsmittel dienen können, bei Temperaturen zwischen -10 und 100 °C, vorzugsweise jedoch bei Temperaturen zwischen 0 und 50 °C, durchgeführt.

Die Acylierung oder Sulfonylierung wird jedoch vorzugsweise mit einem entsprechenden Säurehalogenid oder Säureanhydrid gegebenenfalls in Gegenwart eines säurebindenden Mittels wie vorstehend beschrieben durchgeführt.

h) Zur Herstellung von Verbindungen der allgemeinen Formel I, in der $R_4$ eine Sulfamoylgruppe darstellt: Umsetzung einer Verbindung der allgemeinen Formel

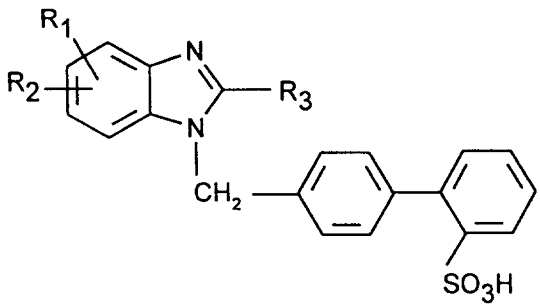

,(X)

in der

$R_1$ bis $R_3$ wie eingangs definiert sind, oder deren reaktionsfähigen Derivaten mit Ammoniak.

Die Umsetzung wird zweckmäßigerweise in einem Lösungsmittel wie Tetrahydrofuran, Chloroform oder Dimethylformamid in Gegenwart eines die Säure aktivierenden Mittels oder eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxybenztriazol, N,N'-Carbonyldiimidazol oder N,N'-Thionyldiimidazol oder Triphenylphosphin/Tetrachlorkohlenstoff, und gegebenenfalls in Gegenwart einer Base wie Natriumcarbonat, Kaliumcarbonat, Kalium-tert.butylat oder 1-Hydroxy-benztriazol/Triethylamin oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100 °C, vorzugsweise jedoch bei Temperaturen zwischen -10 und 80 °C, durchgeführt. Die Umsetzung kann jedoch auch mit einem entsprechenden Säurehalogenid oder Säureanhydrid gegebenenfalls in Gegenwart eines säurebindenden Mittels wie vorstehend beschrieben durchgeführt werden.

i) Zur Herstellung einer Verbindung der allgemeinen Formel I, in der $R_4$ eine Sulfamoylgruppe darstellt: Abspaltung eines Schutzrestes von einer Verbindung der allgemeinen Formel

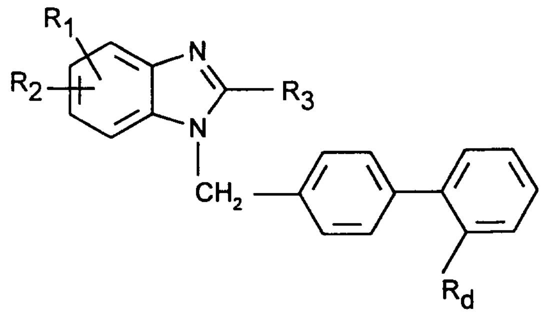

,( XI )

in der
$R_1$ bis $R_3$ wie eingangs definiert sind und
$R_c$ eine mittels Hydrolyse, Aminolyse oder Behandlung mit Säuren in eine Sulfamoylgruppe überführbare Gruppe darstellt.

Die Hydrolyse wird zweckmäßigerweise entweder in Gegenwart einer Säure wie Salzsäure, Schwefelsäure, Phosphorsäure, Trichloressigsäure oder Trifluoressigsäure, oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan und die Aminolyse in Gegenwart eines primären Amins wie Methylamin, Ethylamin oder Propylamin bei Temperaturen zwischen -10 °C und 120 °C, z.B. bei Temperaturen zwischen Raumtemperatur und der Siedetemperatur des Reaktionsgemisches, durchgeführt.

j) Zur Herstellung von Verbindungen der allgemeinen Formel I, in der $R_4$ eine durch eine Alkylcarbonylamino-, Alkoxycarbonylamino-, Alkylaminocarbonylamino-, Dialkylaminocarbonylamino-, Cycloalkylcarbonylamino-, Cycloalkylaminocarbonylamino-, Phenylcarbonylamino-, Phenylaminocarbonylamino-, Phenylalkylcarbonylamino- oder Phenylalkylaminocarbonylaminogruppe substituierte Sulfonylgruppe, in denen der Alkylteil jeweils 1 bis 3 Kohlenstoffatome, der Cycloalkylteil jeweils 5 bis 7 Kohlenstoffatome enthalten und der Phenylkern jeweils durch ein Fluor-, Chlor- oder Bromatom oder durch eine Methoxygruppe substituiert sein kann, darstellt:
Umsetzung einer Verbindung der allgemeinen Formel

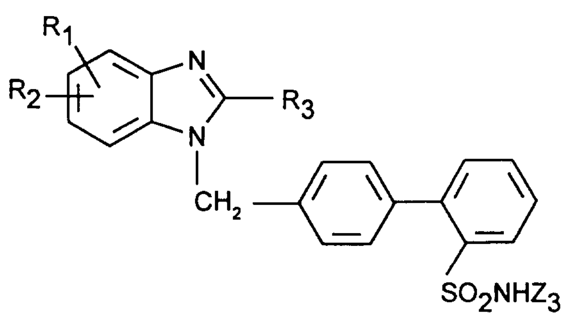

,( XII )

mit einer Verbindung der allgemeinen Formel

$Z_4$-$R_e$ ,  (XIII)

in denen
$R_1$ bis $R_3$ wie eingangs definiert sind,
$Z_3$ eine Alkoxycarbonylgruppe mit insgesamt 2 bis 6 Kohlenstoffatomen,
$Z_4$ ein Wasserstoffatom und
$R_e$ eine Alkylamino-, Dialkylamino-, Cycloalkylamino-, Phenylamino- oder Phenylalkylaminogruppe oder
$Z_3$ ein Wasserstoffatom,
$Z_4$ eine $Z_5$-CO-Gruppe, in der $Z_5$ eine Austrittsgruppe wie ein Halogenatom, z.B. ein Chlor- oder Bromatom, eine Azidogruppe oder eine Acyloxygruppe, z.B. die Acetoxy-, Methoxycarbonyloxy-, Ethoxycarbonyloxy- oder Isobutoxycarbonyloxygruppe, oder auch $Z_5$ zusammen mit dem Wasserstoffatom

einer zur Carbonylgruppe benachbarten Iminogruppe eine weitere Kohlenstoff-Stickstoff-Bindung darstellt, und

$R_e$ eine Alkyl-, Alkoxy-, Alkylamino-, Dialkylamino-, Cycloalkyl-, Cycloalkylamino-, Phenyl-, Phenylamino-, Phenylalkyl- oder Phenylalkylaminogruppe substituierte Sulfonylgruppe, wobei in den vorstehend erwähnten Gruppen der Alkylteil jeweils 1 bis 3 Kohlenstoffatome, der Cycloalkylteil jeweils 5 bis 7 Kohlenstoffatome enthalten und der Phenylkern jeweils durch ein Fluor-, Chlor- oder Bromatom oder durch eine Methoxygruppe substituiert sein kann, bedeuten.

Die Umsetzung wird zweckmäßigerweise in einem Lösungsmittel wie Methanol, Methylenchlorid, Chloroform, Tetrachlorkohlenstoff, Ether, Tetrahydrofuran, Dioxan, Benzol, Toluol, Acetonitril, Sulfolan oder Dimethylformamid gegebenenfalls in Gegenwart einer anorganischen oder organischen Base, gegebenenfalls in Gegenwart eines die Säure aktivierenden Mittels, gegebenenfalls in Gegenwart eines wasserentziehenden Mittels oder gegebenenfalls eines die Aminogruppe aktivierenden Mittels bei Temperaturen zwischen -20 und 200 °C, vorzugsweise jedoch bei Temperaturen zwischen -10 und 160 °C, durchgeführt.

k) Zur Herstellung von Verbindungen der allgemeinen Formel I, in der $R_4$ eine 2,5-Dihydro-5-oxo-1,2,4-oxadiazol-3-yl-Gruppe darstellt:

Umsetzung einer gegebenenfalls im Reaktionsgemisch erhaltenen Verbindung der allgemeinen Formel

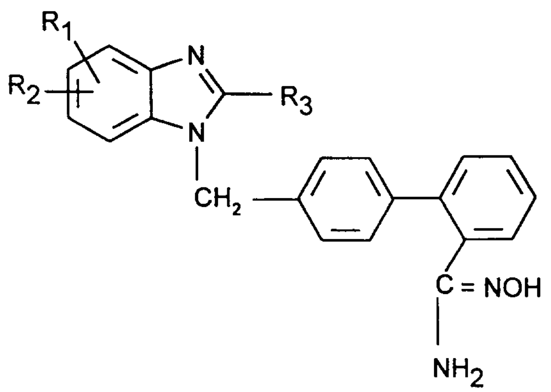

,( XIV )

in der

$R_1$ bis $R_3$ wie eingangs definiert sind, mit einer Verbindung der allgemeinen Formel

$Z_6$-CO-OR$_f$ , (XV)

in der

$Z_6$ eine nukleophile Austrittsgruppe wie ein Halogenatom, z.B. ein Chlor-, Brom- oder Jodatom, und

$R_f$ eine Alkyl-, Aryl- oder Aralkylgruppe, vorzugsweise eine niedere Alkylgruppe wie die Methyl-, Ethyl-, n-Propyl- oder Isopropylgruppe, bedeuten und anschließende Cyclisierung einer so erhaltenen acylierten Amidoxims.

Die Umsetzung wird zweckmäßigerweise in einem Lösungsmittel wie Methylenchlorid, Chloroform, Tetrahydrofuran, Dioxan oder Acetonitril vorzugsweise in Gegenwart einer anorganischen Base wie Natrium oder Kaliumcarbonat oder einer organischen Base wie Triethylamin oder Pyridin, wobei eine tertiäre organische Base gleichzeitig auch als Lösungsmittel verwendet werden kann, bei Temperaturen zwischen 0 und 20 °C durchgeführt.

Die anschließende Cyclisierung einer so erhaltenen acylierten Amidoxims wird zweckmäßigerweise in einem organischen Lösungsmittel wie Benzol, Toluol, Xylol, Tetrahydrofuran oder Dioxan bei erhöhten Temperaturen, z.B. bei Temperaturen zwischen 50 und 100 °C, vorzugsweise bei der Siedetemperatur des verwendeten Lösungsmittel durchgeführt.

Das hierfür erforderliche Amidoxims erhält man zweckmäßigerweise durch Umsetzung eines entsprechenden Nitrils mit Hydroxylamin in Gegenwart eines Lösungsmittels wie Methanol, Ethanol, Methylenchlorid, Chloroform, Dimethylformamid, Tetrahydrofuran oder Dioxan in Gegenwart einer geeigneten Base wie Natriumcarbonat, Kaliumcarbonat, Natriumhydroxid, Triethylamin, Natriummethylat, Natriumethylat oder Natriumhydrid bei Temperaturen zwischen 50 und 100 °C.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Lysin, Methylglucamin, Cyclohexylamin, Äthanolamin, Diäthanolamin und Triäthanolamin in Betracht.

Die als Ausgangsstoffe verwendeten Verbindungen der allgemeinen Formeln II bis XV sind teilweise literaturbekannt (siehe EP-A-0,502,314) oder man erhält diese nach literaturbekannten Verfahren.

So erhält man beispielsweise eine Verbindung der allgemeinen Formel II durch Acylierung eines entsprechenden o-Phenylendiamins und anschließender Cyclisierung oder durch Acylierung einer entsprechenden o-Amino-nitroverbindung, anschließender Reduktion der Nitrogruppe und Cyclisierung, wobei ein so gegebenenfalls erhaltenes NH-Benzimidazol mittels Alkylierung mit einem entsprechenden Biphenylderivat in eine in 1-Stellung entsprechend substituierte Verbindung übergeführt werden kann, und gegebenenfalls anschließende Abspaltung eines verwendeten Schutzrestes.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[(2-n-Propyl-4-methyl-6-(1-methylbenzimidazol-2-yl)-benzimidazol-1-yl)-methyl]-2-(1,3-thiazolidin-2,4-dion-5-methylidinyl)-biphenyl,

B = 4'-[(2-n-Propyl-4-methyl-6-(1-methylbenzimidazol-2-yl)-benzimidazol-1-yl)-methyl]-2-sulfo-biphenyl,

C = 4'-[(2-Ethyl-4-methyl-6-(5,6,7,8-tetrahydro-imidazo[1,2-a]pyridin-2-yl)-benzimidazol-1-yl)-methyl]-2-sulfo-biphenyl,

D = 4'-[(2-n-Propyl-4-methyl-6-(1-methylbenzimidazol-2-yl)-benzimidazol-1-yl)-methyl]-2-trifluoracetylamino-biphenyl,

E = 4'-[(2-Ethyl-4-methyl-6-(5,6,7,8-tetrahydro-imidazo[1,2-a]pyridin-2-yl)-benzimidazol-1-yl)-methyl]-2-trifluoracetylamino-biphenyl,

F = 4'-[(2-n-Propyl-4-methyl-6-(1-methylbenzimidazol-2-yl)-benzimidazol-1-yl)-methyl]-2-(4-methoxy-benzylamino-carbonylaminosulfonyl)-biphenyl,

G = 4'-[(2-n-Propyl-4-methyl-6-(1-methylbenzimidazol-2-yl)-benzimidazol-1-yl)-methyl]-2-(cyclohexylaminocarbonylaminosulfonyl)-biphenyl,

H = 4'-[(2-n-Propyl-4-methyl-6-(1-methylbenzimidazol-2-yl)-benzimidazol-1-yl)-methyl]-2-(benzoylaminosulfonyl)-biphenyl,

I = 4'-[(2-Ethyl-4-methyl-6-(5,6,7,8-tetrahydro-imidazo[1,2-a]pyridin-2-yl)-benzimidazol-1-yl)-methyl]-2-(benzoylaminosulfonyl)-biphenyl,

J = 4'-[(2-n-Butyl-4-methyl-6-(propansultam-1-yl)-benzimidazol-1-yl)-methyl]-2-(benzoylaminosulfonyl)-biphenyl und
K = 4'-[(2-Ethyl-4-methyl-6-(5,6,7,8-tetrahydro-imidazo[1,2-a]pyridin-2-yl)-benzimidazol-1-yl)-methyl]-2-(cyclohexylaminocarbonylaminosulfonyl)-biphenyl
auf ihre biologischen Wirkungen wie folgt untersucht:

Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37 °C mit 50 pM [$^{125}$I]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis H zeigen in dem beschriebenen Test folgende $IC_{50}$-Werte:

| Substanz | $IC_{50}$ [nM] |
|---|---|
| A | 94.0 |
| B | 8.0 |
| C | 3.4 |
| D | 40.0 |
| E | 28.0 |
| F | 110.0 |
| G | 310.0 |
| H | 78.0 |
| I | 36.0 |
| J | 13.9 |
| K | 73.0 |

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, der Bulimie, sowie von Störungen kognitiver Funktionen.

Die zur Erzielung einer entsprechenden Wirkung am Erwachsenen erforderliche Dosierung beträgt zweckmäßigerweise bei intravenöser Gabe 0,5 bis 100 mg, vorzugsweise 1 bis 70 mg, und bei oraler Gabe 0,1 bis 200 mg, vorzugsweise 1 bis 100 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, ACE-Hemmer, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten,

Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

## Beispiel 1

### 4'-[(2-n-Propyl-4-methyl-6-(1-methylbenzimidazol-2-yl)-benzimidazol-1-yl)-methyl]-2-hydroxymethyl-biphenyl

5,4 g 4'-[(2-n-Propyl-4-methyl-6-(1-methylbenzimidazol-2-yl) benzimidazol-1-yl)-methyl]-2-tert.butoxycarbonyl-biphenyl in 100 ml Tetrahydrofuran werden bei Raumtemperatur zu 0,7 g Lithiumaluminiumhydrid in 100 ml Tetrahydrofuran gegeben und 2 Stunden bei Raumtemperatur gerührt. Anschließend wird auf Eiswasser gegossen, mit Essigester extrahiert und eingedampft. Nach Säulenchromatographie an Kieselgel mit Dichlormethan/Ethanol (97:3) erhält man das gewünschte Produkt, das roh weiter verarbeitet wird.

Ausbeute: 2,9 g.

$R_f$-Wert: 0,5 g (Kiselgel; Essigester/Ethanol/Ammoniak = 90:10:1)

## Beispiel 2

### 4-[(2-n-Propyl-4-methyl-6-(1-methylbenzimidazol-2-yl)-benzimidazol-1-yl)-methyl]-2-formyl-biphenyl

2 g 4'-[(2-n-Propyl-4-methyl-6-(1-methylbenzimidazol-2-yl)-benzimidazol-1-yl)-methyl]-2-hydroxymethyl-biphenyl werden in 25 ml Dichlormethan mit 2.5 g Mangandioxid versetzt und bei Raumtemperatur 12 Stunden gerührt. Anschließend wird über Kieselgur filtriert und mit Dichlormethan nachgewaschen. Nach Eindampfen erhält man 1.84 g als hellgelben Schaum.

$R_f$-Wert: 0,6 (Kieselgel; Essigester/Ethanol/Ammoniak = 90:10:1)

## Beispiel 3

### 4'-[(2-n-Propyl-4-methyl-6-(1-methylbenzimidazol-2-yl)benzimidazol-1-yl)-methyl]-2-(1,3-thiazolidin-2,4-dion-5-methylidinyl)-biphenyl

1,8 g 4-[(2-n-Propyl-4-methyl-6-(1-methylbenzimidazol-2-yl)-benzimidazol-1-yl)-methyl]-2-formyl-biphenyl, gelöst in 15 ml Eisessig, werden mit 0,62 g Thiazolidin-2,4-dion auf 120°C erhitzt und bei dieser Temperatur 12 Stunden gerührt. Nach dem Eindampfen wird an Kieselgel mit Dichlormethan/Ethanol chromatographiert (17:1). Anschließend werden 1,4 g des eluierten Produkts in 50 ml Ethanol gelöst und mit 10 ml 2n Natronlauge versetzt. Nach 1-stündigem Rühren bei Raumtemperatur wird mit 40 ml Wasser versetzt und der Alkohol im Vakuum abgedampft. Das Produkt wird mit Essigester extrahiert, über Natriumsulfat getrocknet und eingedampft.

Ausbeute: 1,1 g,

Schmelzpunkt: 252-254°C.

## Beispiel 4

### 4'-[(2-n-Propyl-4-methyl-6-(1-methylbenzimidazol-2-yl)-benzimidazol-1-yl)-methyl]-2-tert.butoxycarbonylamino-biphenyl

12,4 g 4'-[(2-n-Propyl-4-methyl-6-(1-methylbenzimidazol-2-yl)-benzimidazol-1-yl)-methyl]-2-carboxy-biphenyl, gelöst in 150 ml Chloroform, werden mit 3,5 ml Triethylamin versetzt und anschließend bei 0°C 3 ml Chlorameisensäure-ethylester zugegeben. Nach 1-stündigem Rühren bei 0°C werden 0,2 g Tetrabutylammoniumbromid und dann 2,4 g Natriumazid in 8,5 ml Wasser zugetropft. Nach 1 Stunde werden jeweils 50 ml Chloroform und Wasser zugegeben, die Chloroform-Phase über Natriumsulfat getrocknet und eingedampft. Das Produkt wird mit tert.Butanol aufgenommen und 3 Stunden am Rückfluß gekocht. Nach dem Eindampfen erhält man 4,8 g Rohprodukt, das an Kieselgel mit Dichlormethan/Ethanol (19:1) chromatographiert wird.
Ausbeute: 3,1 g,
Schmelzpunkt: 182-184 °C.

Beispiel 5

4'-[(2-n-Propyl-4-methyl-6-(1-methylbenzimidazol-2-yl)-benzimidazol-1-yl)-methyl]-2-amino-biphenyl

10,3 g 4'-[(2-n-Propyl-4-methyl-6-(1-methylbenzimidazol-2-yl)-benzimidazol-1-yl)-methyl]-2-tert.butoxycarbonylamino-biphenyl werden in 50 ml Dichlormethan mit 10 ml Trifluoressigsäure 1 Stunde am Rückfluß gekocht. Nach dem Abkühlen wird mit gesättigter Natriumbicarbonat-Lösung neutralisiert, die Dichlormethan-Phase über Natriumsulfat getrocknet und eingedampft.
Ausbeute: 8,2 g,
Schmelzpunkt: 208-210 °C (Aceton).

Beispiel 6

4'-[(2-n-Propyl-4-methyl-6-(1-methylbenzimidazol-2-yl)-benzimidazol-1-yl)-methyl]-2-sulfo-biphenyl

9,0 g 4'-[(2-n-Propyl-4-methyl-6-(1-methylbenzimidazol-2-yl)-benzimidazol-1-yl)-methyl]-2-amino-biphenyl werden in 170 ml 6N Salzsäure suspendiert und bei 0-5 °C innerhalb 1 Stunde 1,95 g Natriumnitrit in 30 ml Wasser zugetropft. Anschließend wird noch 2 Stunden bei 0 °C gerührt und mit Harnstoff versetzt. Die entstehende Lösung wird bei 0-5 °C zu einer Mischung, bestehend aus 40 ml einer gesättigten Lösung von Schwefeldioxid in Eisessig und 2,25 g Kupfer(II)chlorid-monohydrat in 3,3 ml Wasser, zugetropft. Anschließend wird noch 2 Stunden bei Raumtemperatur gerührt, unter Kühlung mit konzentriertem Ammoniak alkalisch gestellt und das ausgefallene Produkt abgesaugt. Das Filtrat wird mit Essigester ausgeschüttelt und die Essigester-Phase mit dem ausgefallenen Produkt eingeengt. Nach Chromatographie an Kieselgel mit Dichlormethan/Ethanol (19:1) erhält man 4,2 g vom Schmelzpunkt 307-310 °C.

Beispiel 7

4'-[(2-Ethyl-4-methyl-6-(5,6,7,8-tetrahydro-imidazo[1,2-a]pyridin-2-yl)-benzimidazol-1-yl)-methyl]-2-sulfo-biphenyl

Hergestellt analog den Beispielen 4 bis 6 aus 4'-[(2-Ethyl-4-methyl-6-(5,6,7,8-tetrahydro-imidazo[1,2-a]-pyridin-2-yl)-benzimidazol-1-yl)-methyl]-2-carboxy-biphenyl.
Schmelzpunkt: >330 °C.

Beispiel 8

4'-[(2-n-Propyl-4-methyl-6-(1-methylbenzimidazol-2-yl)-benzimidazol-1-yl)-methyl]-2-trifluoracetylamino-biphenyl

Zu 0,5 g 4'-[(2-n-Propyl-4-methyl-6-(1-methylbenzimidazol-2-yl)-benzimidazol-1-yl)-methyl]-2-amino-biphenyl in 25 ml Dichlormethan und 0,5 ml Triethylamin werden bei -50 °C 0,5ml Trifluoressigsäureanhydrid getropft. Anschließend wird die Mischung auf Raumtemperatur erwärmt und 4 Stunden bei dieser Temperatur gerührt. Dann wird mit Wasser gewaschen, über Natriumsulfat getrocknet und eingedampft. Nach Chromatographie an Kieselgel mit Dichlormethan/Ethanol (50:1) erhält man 0,4 g vom
Schmelzpunkt 115-120 °C.

Beispiel 9

4'-[(2-Ethyl-4-methyl-6-(5,6,7,8-tetrahydro-imidazo[1,2-a]pyridin-2-yl)-benzimidazol-1-yl)-methyl)-2-trifluoracetylaminobiphenyl

Hergestellt analog Beispiel 8 aus 4'-[(2-Ethyl-4-methyl-6-(5,6,7,8-tetrahydro-imidazo[1,2-a]-pyridin-2-yl)-benzimidazol-1-yl)-methyl]-2-amino-biphenyl.
Schmelzpunkt: 246-248 °C.

Beispiel 10

4'-[(2-n-Propyl-4-methyl-6-(1-methylbenzimidazol-2-yl)-benzimidazol-1-yl)-methyl]-2-sulfamoyl-biphenyl

1,2 g 4'-[(2-n-Propyl-4-methyl-6-(1-methylbenzimidazol-2-yl)-benzimidazol-1-yl)-methyl]-2-sulfo-biphenyl werden mit 10 ml Pyridin 1 Stunde bei 60 °C gerührt. Die Lösung wird dann eingedampft, der Rückstand mit Aceton verrührt und abgesaugt. Anschließend wird in 15 ml Thionylchlorid aufgenommen und 1 Stunde bei 60 °C gerührt. Nach dem Eindampfen wird der Rückstand in 10 ml Dimethylformamid aufgenommen und in 10 ml Dimethylformamid,gesättigt mit Ammoniak, eingerührt. Nach 15-minütigem Rühren bei Raumtemperatur wird eingedampft und an Kieselgel mit Dichlormethan/Ethanol (98:2) chromatographiert.
Ausbeute: 0,4 g,
Schmelzpunkt: 143-145 °C.

Beispiel 11

4'-[(2-n-Propyl-4-methyl-6-(1-methylbenzimidazol-2-yl)-benzimidazol-1-yl)-methyl]-2-ethoxycarbonylaminosulfonyl-biphenyl

0,3 g 4'-[(2-n-Propyl-4-methyl-6-(1-methylbenzimidazol-2-yl)-benzimidazol-1-yl)-methyl]-2-sulfamoyl-biphenyl, 170 mg Kaliumcarbonat, 5 ml Ethylenglykoldimethylether und 0,11 ml Chlorameisensäure-ethylester werden 1 Stunde am Rückfluß gekocht. Anschließend wird mit 30 ml Essigester und 15 ml 10%iger Kaliumdihydrogenphosphat-Lösung versetzt, die Essigester-Phase über Natriumsulfat getrocknet und eingedampft. Das erhaltene Produkt wird roh weiterverarbeitet.
$R_f$-Wert: 0,53 (Kieselgel; Methylenchlorid/Ethanol = 19:1)

Beispiel 12

4'-[(2-n-Propyl-4-methyl-6-(1-methylbenzimidazol-2-yl)-benzimidazol-1-yl)-methyl]-2-(4-methoxybenzylaminocarbonylaminosulfonyl)-biphenyl

0,37 g 4'-[(2-n-Propyl-4-methyl-6-(1-methylbenzimidazol-2-yl)-benzimidazol-1-yl)-methyl]-2-ethoxycarbonylaminosulfonyl-biphenyl werden in 5 ml Toluol mit 0,1 ml 4-Methoxybenzylamin 18 Stunden auf 90 °C erhitzt. Anschließend wird eingedampft und an Kieselgel mit Dichlormethan/Ethanol (100:2) chromatographiert.
Ausbeute: 0,15 g,
Schmelzpunkt: 150-154 °C.

Beispiel 13

4'-[(2-n-Propyl-4-methyl-6-(1-methylbenzimidazol-2-yl)-benzimidazol-1-yl)-methyl]-2-(cyclohexylaminocarbonylaminosulfonyl)-biphenyl

0,275 g 4'-[(2-n-Propyl-4-methyl-6-(1-methylbenzimidazol-2-yl)-benzimidazol-1-yl)-methyl]-2-sulfamoyl-biphenyl werden in 2 ml Pyridin mit 0,5 ml Cyclohexylisocyanat 20 Stunden am Rückfluß gekocht. Anschließend wird eingedampft, der Rückstand in Aceton aufgeschlämmt und der unlösliche Feststoff abfiltriert. Nach dem Eindampfen des Filtrats wird das so erhaltene Rohprodukt durch Säulenchromatographie über Kieselgel (Laufmittel: Dichlormethan/Ethanol = 50:1) gereinigt.
Ausbeute: 0,14 g,
Schmelzpunkt: 174-176 °C.

Beispiel 14

4'-[(2-n-Propyl-4-methyl-6-(1-methylbenzimidazol-2-yl)-benzimidazol-1-yl)-methyl]-2-(N,N-dimethylaminocarbonylaminosulfonyl)-biphenyl

1,82 g 2-n-Propyl-4-methyl-6-(1-methylbenzimidazol-2-yl)-benzimidazol, gelöst in 100 ml Dimethylsulfoxid, werden mit 0,74 g Kalium-tert.butylat versetzt und 0,5 Stunden bei Raumtemperatur gerührt. Anschließend werden 3,05 g 4'-Brommethyl-2-(N,N-dimethylaminocarbonylsulfonamid)-biphenyl (hergestellt analog EP-A-0,503,162) zugegeben und 16 Stunden bei 50 °C gerührt. Nach Zugabe von weiteren 1,5 g der Brommethyl-Verbindung wird noch weitere 8 Stunden bei 50 °C gerührt. Dann wird mit Essigester und 10%iger Natriumchlorid-Lösung versetzt, dreimal mit Essigester ausgeschüttelt, die organische Phase mit Natriumchlorid-Lösung gewaschen und über Natriumsulfat getrocknet. Nach dem Eindampfen wird über Kieselgel mit Dichlormethan/Ethanol (9:1) chromatographiert.
Ausbeute: 1,9 g,
$R_f$-Wert: 0,55 (Kieselgel; Methylenchlorid/Ethanol = 50:1)

Beispiel 15

4'-[(2-n-Propyl-4-methyl-6-(1-methylbenzimidazol-2-yl)-benzimidazol-1-yl)-methyl]-2-sulfamoyl-biphenyl

1,9 g 4'-[(2-n-Propyl-4-methyl-6-(1-methylbenzimidazol-2-yl)-benzimidazol-1-yl)-methyl]-2-(N,N-dimethylaminocarbonylaminosulfonyl)-biphenyl werden in 50 ml Ethanol mit 25 ml 40%iger Methylamin-Lösung 2 Stunden bei Raumtemperatur gerührt und anschließend eingedampft. Das erhaltene Produkt wird roh weiterverarbeitet.
Ausbeute: 1,1 g,
$R_f$-Wert: 0,47 (Kieselgel; Methylenchlorid/Ethanol = 50:1)

Beispiel 16

4'-[(2-n-Propyl-4-methyl-6-(1-methylbenzimidazol-2-yl)-benzimidazol-1-yl)-methyl]-2-(benzoylaminosulfonyl)-biphenyl

145 mg Benzoesäure und 192 mg Carbonyldiimidazol werden in 1 ml Tetrahydrofuran bei 50 °C 2 Stunden gerührt. Dazu wird eine Lösung aus 163 mg 4'-[(2-n-Propyl-4-methyl-6-(1-methylbenzimidazol-2-yl)-benzimidazol-1-yl)-methyl]-2-sulfamoyl-biphenyl, 0,133 ml 1,8-Diazabicyclo[5.4.0]undec-7-en und 1 ml Tetrahydrofuran gegeben und 2,5 Stunden bei 55 °C gehalten. Anschließend wird mit 50 ml Essigester und 20 ml 5%iger Zitronensäure versetzt. Die organische Phase wird mit Natriumsulfat getrocknet und eingedampft. Der erhaltene Rückstand wird über Kieselgel mit Dichlormethan/Ethanol (95:5) chromatographiert.
Ausbeute: 157 mg (amorph),
Massenspektrum: $M^+$ = 653
Analog den Beispielen 14 bis 16 werden folgende Verbindungen hergestellt:

(1) 4'-[(2-Ethyl-4-methyl-6-(5,6,7,8-tetrahydro-imidazo[1,2-a]pyridin-2-yl)-benzimidazol-1-yl)-methyl]-2-(benzoylaminosulfonyl)-biphenyl
Hergestellt aus 2-Ethyl-4-methyl-6-(5,6,7,8-tetrahydro-imidazo[1,2-a]pyridin-2-yl)-benzimidazol Massenspektrum: $M^+$ = 629

(2) 4'-[(2-n-Butyl-4-methyl-6-(propansultam-1-yl)-benzimidazol-1-yl)-methyl]-2-(benzoylaminosulfonyl)-biphenyl
Hergestellt aus 2-n-Butyl-4-methyl-6-(propansultam-1-yl)-benzimidazol Massenspektrum: $(M+H)^+$ = 657

(3) 4'-[[2-n-Propyl-4-methyl-6-[1-(2-morpholinoethyl)-imidazol-4-yl]-benzimidazol-1-yl]-methyl]-2-(benzoylaminosulfonyl)-biphenyl
Hergestellt aus 2-n-Propyl-4-methyl-6-[1-(2-morpholinoethyl)-imidazol-4-yl]-benzimidazol
Massenspektrum: $M^+$ = 702

Beispiel 17

4'-[(2-Ethyl-4-methyl-6-(5,6,7,8-tetrahydro-imidazo[1,2-a]pyridin-2-yl)-benzimidazol-1-yl)-methyl]-2-(cyclohexylaminocarbonylaminosulfonyl)-biphenyl

0,25 g 4'-[(2-Ethyl-4-methyl-6-(5,6,7,8-tetrahydro-imidazo[1,2-a]pyridin-2-yl)-benzimidazol-1-yl)-methyl]-2-sulfamoyl-biphenyl in 2 ml Pyridin werden mit 0,5 ml Cyclohexylisocyanat 6 Stunden am Rückfluß gekocht. Nach dem Eindampfen wird an Kieselgel mit Dichlormethan/Ethanol (95:5) chromatographiert.
Ausbeute: 85 mg,
Massenspektrum: $M^+$ = 651

Beispiel 18

4'-[[2-n-Propyl-4-methyl-6-(1-methylbenzimidazol-2-yl)-1H-benzimidazol-1-yl]-methyl]-2-(hydroxycarbamidoyl)-biphenyl

Zu einer Lösung von 6,90 g Hydroxylamin-hydrochlorid in 50 ml Dimethylsulfoxid werden 1,35 g Natriummethylat und 3,5 g 4'-[[2-n-Propyl-4-methyl-6-(1-methyl-benzimidazol-2-yl)-1H-benzimidazol-1-yl]-methyl]-2-cyano-biphenyl gegeben. Das Reaktionsgemisch wird 20 Stunden bei 90°C gerührt und nach Abkühlung mit Eiswasser hydrolysiert. Der gebildete Niederschlag wird abgesaugt, mit Wasser gewaschen und getrocknet. Das erhaltene Rohprodukt wird an Kieselgel chromatographiert, wobei als Laufmittel anfangs Essigester, später Essigester/Ethanol/Ammoniak (19:1:0,1) verwendet wird. Die einheitlichen Fraktionen werden vereinigt, eingedampft, mit Ether verrieben und getrocknet.
Ausbeute: 0,99 g (27 % der Theorie),
Schmelzpunkt: 185-187°C

Beispiel 19

4'-[[2-n-Propyl-4-methyl-6-(1-methylbenzimidazol-2-yl)-1H-benzimidazol-1-yl]-methyl]-2-(2,5-dihydro-5-oxo-1,2,4-oxadiazol-3-yl)-biphenyl

Zu einer Suspension von 960 mg 4'-[[2-n-Propyl-4-methyl-6-(1-methylbenzimidazol-2-yl)-1H-benzimidazol-1-yl]-methyl]-2-(hydroxycarbamidoyl)-biphenyl und 200 mg Triethylamin in 40 ml werden bei 0°C 220 mg Chlorameisensäure-ethylester zugetropft. Anschließend wird das Reaktionsgemisch 6 Stunden zum Rückfluß erhitzt. Nach Abkühlung wird das Reaktionsgemisch mit Methylenchlorid versetzt, mit Wasser gewaschen und getrocknet. Das erhaltene Rohprodukt wird an Kieselgel chromatographiert, wobei als Laufmittel anfangs Methylenchlorid, später Methylenchlorid/Ethanol (50:1, 25:1 und 19:1) verwendet wird. Die einheitlichen Fraktionen werden vereinigt, eingedampft, mit Ether verrieben und getrocknet.
Ausbeute: 0,27 g (27 % der Theorie),
Schmelzpunkt: 266-268°C
Massenspektrum: $M^+$ = 555

Beispiel 20

4'-[[2-n-Propyl-4-methyl-6-(1-methyl-4,5,6,7-tetrahydro-benzimidazol-2-yl)-1H-benzimidazol-1-yl]-methyl]-2-(hydroxycarbamidoyl)-biphenyl

Hergestellt analog Beispiel 18 aus 4'-[[2-n-Propyl-4-methyl-6-(1-methyl-4,5,6,7-tetrahydro-benzimidazol-2-yl)-1H-benzimidazol-1-yl]-methyl]-2-cyano-biphenyl und Hydroxylamin/Natriummethanolat.
Ausbeute: 25 % der Theorie,
Schmelzpunkt: 221-224°C

Beispiel 21

4'-[[2-n-Propyl-4-methyl-6-(1-methyl-4,5,6,7-tetrahydro-benzimidazol-2-yl)-1H-benzimidazol-1-yl]-methyl]-2-(2,5-dihydro-5-oxo-1,2,4-oxadiazol-3-yl)-biphenyl

Hergestellt analog Beispiel 19 aus 4'-[[2-n-Propyl-4-methyl-6-(1-methyl-4,5,6,7-tetrahydro-benzimidazol-2-yl)-1H-benzimidazol-1-yl]-methyl]-2-(hydroxycarbamidoyl)-biphenyl und Chlorameisensäure-ethylester/Triethylamin.
Ausbeute: 55 % der Theorie,
Schmelzpunkt: ab 199 °C (Zers.)
Massenspektrum: $M^+$ = 558
Bei den nachfolgenden pharmazeutischen Anwendungsbeispielen kann als Wirksubstanz jede geeignete Verbindung der Formel I eingesetzt werden:

Beispiel I

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpreßt.

Beispiel II

| Dragées, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1.0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel III

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1.2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 °C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel IV

| Kapseln, enthaltend 250 mg Wirkstoff | |
|---|---|
| Wirkstoff | 250,0 mg |
| Maisstärke | 68,5 mg |
| Magnesiumstearat | 1.5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel V

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 ° C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VI

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40 ° C wird die gemahlene Wirksubstanz in der Schmelze homogen dispergiert. Es wird auf 38 ° C abgekühlt und in schwach vorgekühlte Suppositorienformen ausgegossen.

**Patentansprüche**

**1.** Benzimidazole der allgemeinen Formel

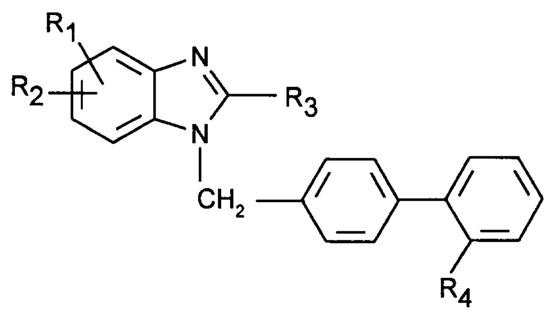

,(I)

in der
$R_1$ ein Fluor-, Chlor- oder Bromatom, eine Alkylgruppe mit 1 bis 3 Kohlenstoffatomen oder eine Trifluormethylgruppe,
$R_2$ eine über ein Kohlenstoffatom gebundene 5-gliedrige Heteroarylgruppe, die eine Iminogruppe, ein Sauerstoff- oder Schwefelatom oder eine Iminogruppe und ein Sauerstoff-, Schwefel- oder Stickstoffatom enthält, und an die über zwei benachbarte Kohlenstoffatome eine n-Propylen-, n-Butylen- oder 1,3-Butadienylenbrücke oder über eine Iminogruppe und ein benachbartes Kohlenstoffatom eine n-Butylen- oder 1,3-Butadienylenbrücke angefügt sein kann, wobei
das Kohlenstoffgerüst der vorstehend erwähnten bicyclischen Ringe durch ein Fluor-, Chlor- oder Bromatom, durch eine Trifluormethyl-, Alkyl-, Alkoxy-, Cyano-, Aminocarbonyl-, Carboxy-, Nitro-, Amino-, Alkylamino- oder Dialkylaminogruppe mit jeweils 1 bis 3 Kohlenstoffatomen im Alkyl- und Alkoxyteil mono- oder disubstituiert sein kann, wobei die Substituenten gleich oder verschieden sein können, und zusätzlich eine HN-Gruppe der vorstehend erwähnten heteroaromatischen Ringe durch eine Alkylgruppe mit 1 bis 6 Kohlenstoffatomen, durch eine Phenylalkylgruppe mit 1 bis 3 Kohlenstoffatomen im Alkylteil oder durch eine Cycloalkylgruppe mit 3 bis 6 Kohlenstoffatomen substituiert sein kann,
eine durch eine Alkylgruppe mit 1 bis 3 Kohlenstoffatomen substituierte Imidazol-4-yl-Gruppe, in der der Alkylteil in 2- oder 3-Stellung durch eine Dimethylamino-, Diethylamino-, Pyrrolidino-, Piperidino-, Hexamethylenimino- oder Morpholinogruppe substituiert sein kann,
eine Pyrrolidino-, Piperidino- oder Hexamethyleniminogruppe, in denen jeweils eine Methylengruppe

durch eine Carbonyl- oder Sulfonylgruppe ersetzt ist,
eine gegebenenfalls durch eine Alkylgruppe mit 1 bis 3 Kohlenstoffatomen oder durch eine Phenylgruppe substituierte Maleinsäureimidogruppe oder
eine gegebenenfalls durch eine Alkylgruppe mit 1 bis 3 Kohlenstoffatomen substituierte Imidazolidin-2,4-dion-3-yl-Gruppe,
$R_3$ eine Alkylgruppe mit 1 bis 4 Kohlenstoffatomen, eine Cycloalkylgruppe mit 3 bis 5 Kohlenstoffatomen, eine Alkoxy- oder Alkylthiogruppe mit jeweils 1 bis 3 Kohlenstoffatomen im Alkylteil und
$R_4$ eine gegebenenfalls durch eine Alkoxycarbonylgruppe mit insgesamt 2 bis 6 Kohlenstoffatomen, durch eine Trifluoracetyl- oder Trifluormethylsulfonylgruppe substituierte Aminogruppe,
eine durch eine Hydroxy-, Amino-, Alkylcarbonylamino-, Alkoxycarbonylamino-, Alkylaminocarbonylamino-, Dialkylaminocarbonylamino-, Cycloalkylcarbonylamino-, Cycloalkylaminocarbonylamino-, Phenylcarbonylamino-, Phenylaminocarbonylamino-, Phenylalkylcarbonylamino- oder Phenylalkylaminocarbonylaminogruppe substituierte Sulfonylgruppe, in denen der Alkylteil jeweils 1 bis 3 Kohlenstoffatome, der Cycloalkylteil jeweils 5 bis 7 Kohlenstoffatome enthalten und der Phenylkern jeweils durch ein Fluor-, Chlor- oder Bromatom oder durch eine Methoxygruppe substituiert sein kann,
eine Hydroxycarbamidoyl-, Thiazolidin-2,4-dion-5-methyliden- oder 2,5-Dihydro-5-oxo-oxadiazol-3-yl-Gruppe bedeuten,
deren Tautomere und deren Salze.

**2.** Benzimidazole der allgemeinen Formel I gemäß Anspruch 1, in der
$R_1$ eine Methylgruppe,
$R_2$ eine gegebenenfalls in 1-Stellung durch eine Methylgruppe substituierte Benzimidazol-2-yl-Gruppe, eine gegebenenfalls in 1-Stellung durch eine Alkylgruppe mit 1 bis 3 Kohlenstoffatomen subsituierte Imidazol-4-yl-Gruppe, in der der Alkylsubstituent in 2- oder 3-Stellung durch eine Morpholinogruppe substituiert sein kann, eine 5,6,7,8-Tetrahydro-imidazo[1,2-a]pyridin-2-yl-, Propansultam-1-yl- oder Butansultam-1-yl-Gruppe,
$R_3$ eine geradkettige Alkylgruppe mit 2 bis 4 Kohlenstoffatomen und
$R_4$ eine Aminogruppe, eine durch eine Hydroxy-, Amino-, Dimethylaminocarbonylamino-, Phenylcarbonylamino-, Cycloalkylaminocarbonylamino- oder Benzylaminocarbonylaminogruppe substituierte Sulfonylgruppe, in denen der Cycloalkylteil jeweils 5 oder 6 Kohlenstoffatome enthalten und der Phenylteil jeweils durch eine Methoxygruppe substituiert sein kann, eine Trifluoracetylamino-, tert.Butoxycarbonylamino-, Trifluormethylsulfonylamino-, 1,3-Thiazolidin-2,4-dion-5-methyliden- oder 1,2,4-Oxadiazol-5-on-3-yl-Gruppe bedeuten,
deren Tautomere und deren Salze.

**3.** Benzimidazole der allgemeinen Formel I gemäß Anspruch 1, in der
$R_1$ in 4-Stellung eine Methylgruppe,
$R_2$ in 6-Stellung eine gegebenenfalls in 1-Stellung durch eine Methylgruppe substituierte Benzimidazol-2-yl-Gruppe, eine in 1-Stellung durch eine Alkylgruppe mit 1 bis 3 Kohlenstoffatomen subsituierte Imidazol-4-yl-Gruppe, in der der Alkylsubstituent in 2- oder 3-Stellung durch eine Morpholinogruppe substituiert sein kann, eine 5,6,7,8-Tetrahydro-imidazo[1,2-a]pyridin-2-yl-, Propansultam-1-yl- oder Butansultam-1-yl-Gruppe,
$R_3$ eine geradkettige Alkylgruppe mit 2 bis 4 Kohlenstoffatomen und
$R_4$ eine durch eine Hydroxy-, Amino-, Dimethylaminocarbonylamino-, Phenylcarbonylamino-, Cycloalkylaminocarbonylamino- oder Benzylaminocarbonylaminogruppe substituierte Sulfonylgruppe, in denen der Cycloalkylteil jeweils 5 oder 6 Kohlenstoffatome enthalten und der Phenylteil jeweils durch eine Methoxygruppe substituiert sein kann, eine Trifluoracetylamino-, Trifluormethylsulfonylamino-, 1,3-Thiazolidin-2,4-dion-5-methyliden- oder 1,2,4-Oxadiazol-5-on-3-yl-Gruppe bedeuten, und
deren Salze.

**4.** Benzimidazol der allgemeinen Formel I gemäß Anspruch 1, in der
$R_1$ in 4-Stellung eine Methylgruppe,
$R_2$ in 6-Stellung eine 1-Methyl-benzimidazol-2-yl-, 1-(2-Morpholinoethyl)-imidazol-4-yl-, 5,6,7,8-Tetrahydro-imidazo[1,2-a]pyridin-2-yl- oder Butansultam-1-yl-Gruppe,
$R_3$ eine Ethyl- oder n-Propylgruppe und
$R_4$ eine durch eine Hydroxy-, Dimethylaminocarbonylamino-, Phenylcarbonylamino-, Cycloalkylaminocarbonylamino- oder Benzylaminocarbonylaminogruppe substituierte Sulfonylgruppe, in denen der Cycloalkylteil jeweils 5 oder 6 Kohlenstoffatome enthalten und der Phenylteil jeweils durch eine

Methoxygruppe substituiert sein kann, eine Trifluoracetylamino-, Trifluormemehylsulfonylamino-, 1,3-Thiazolidin-2,4-dion-5-methyliden- oder 1,2,4-Oxadiazol-5-on-3-yl-Gruppe bedeuten, und deren Salze.

**5.** Folgende Benzimidazole der allgemeinen Formel I:
(a) 4'-[(2-n-Propyl-4-methyl-6-(1-methylbenzimidazol-2-yl)-benzimidazol-1-yl)-methyl]-2-(1,3-thiazolidin-2,4-dion-5-methylidinyl)-biphenyl,
(b) 4'-[(2-n-Propyl-4-methyl-6-(1-methylbenzimidazol-2-yl)-benzimidazol-1-yl)-methyl]-2-sulfo-biphenyl,
(c) 4'-[(2-Ethyl-4-methyl-6-(5,6,7,8-tetrahydro-imidazo[1,2-a]pyridin-2-yl)-benzimidazol-1-yl)-methyl]-2-sulfo-biphenyl,
(d) 4'-[(2-n-Propyl-4-methyl-6-(1-methylbenzimidazol-2-yl)-benzimidazol-1-yl)-methyl]-2-trifluoracetylamino-biphenyl,
(e) 4'-[(2-Ethyl-4-methyl-6-(5,6,7,8-tetrahydro-imidazo[1,2-a]pyridin-2-yl)-benzimidazol-1-yl)-methyl]-2-trifluoracetylaminobiphenyl,
(f) 4'-[(2-n-Propyl-4-methyl-6-(1-methylbenzimidazol-2-yl)-benzimidazol-1-yl)-methyl]-2-(4-methoxybenzylaminocarbonylaminosulfonyl)-biphenyl,
(g) 4'-[(2-n-Propyl-4-methyl-6-(1-methylbenzimidazol-2-yl)-benzimidazol-1-yl)-methyl]-2-(cyclohexylaminocarbonylaminosulfonyl)-biphenyl,
(h) 4'-[(2-n-Propyl-4-methyl-6-(1-methylbenzimidazol-2-yl)-benzimidazol-1-yl)-methyl]-2-(benzoylaminosulfonyl)-biphenyl,
(i) 4'-[(2-Ethyl-4-methyl-6-(5,6,7,8-tetrahydro-imidazo[1,2-a]pyridin-2-yl)-benzimidazol-1-yl)-methyl]-2-(benzoylaminosulfonyl)-biphenyl,
(j) 4'-[(2-n-Butyl-4-methyl-6-(propansultam-1-yl)-benzimidazol-1-yl)-methyl]-2-(benzoylaminosulfonyl)-biphenyl und
(k) 4'-[(2-Ethyl-4-methyl-6-(5,6,7,8-tetrahydro-imidazo[1,2-a]pyridin-2-yl)-benzimidazol-1-yl)-methyl]-2-(cyclohexylaminocarbonylaminosulfonyl)-biphenyl
sowie deren Salze.

**6.** 4'-[(2-Ethyl-4-methyl-6-(5,6,7,8-tetrahydro-imidazo[1,2-a]pyridin-2-yl)-benzimidazol-1-yl)-methyl]-2-(cyclohexylaminocarbonylaminosulfonyl)-biphenyl
und deren Salze.

**7.** Physiologisch verträgliche Salze der Verbindungen nach mindestens einem der Ansprüche 1 bis 6 mit anorganischen oder organischen Säuren oder Basen.

**8.** Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels mit Angiotensin-antagonistischer Wirkung.

**10.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

**11.** Verfahren zur Herstellung der Benzimidazole gemäß den Ansprüchen 1 bis 7, dadurch gekennzeichnet, daß
a) ein Benzimidazol der allgemeinen Formel

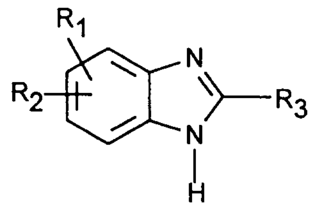

,( II )

in der
$R_1$ bis $R_3$ wie in den Ansprüchen 1 bis 6 definiert sind, mit einer Biphenylverbindung der allgemeinen Formel

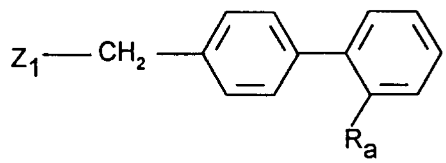

,( III )

in der
$R_a$ die für $R_4$ in den Ansprüchen 1 bis 6 erwähnten Bedeutungen besitzt, wobei jedoch ein vorhandenes reaktives Wasserstoffatom durch einen üblichen Schutzrest geschützt ist und
$Z_1$ eine nukleophile Austrittsgruppe darstellt, umgesetzt und anschließend ein gegebenenfalls verwendeter Schutzrest abgespalten wird oder
b) zur Herstellung von Verbindungen der allgemeinen Formel I, in der $R_4$ eine Hydroxymethylgruppe darstellt, eine Verbindung der allgemeinen Formel

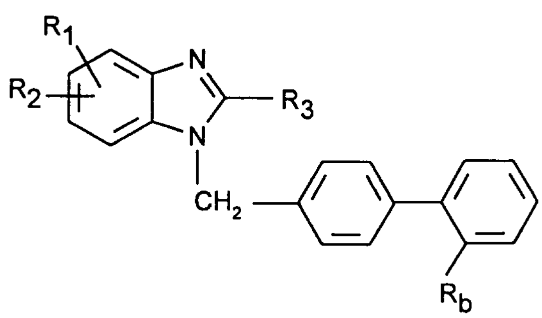

,( IV )

in der
$R_1$ bis $R_3$ wie in den Ansprüchen 1 bis 6 definiert sind und $R_b$ eine veresterte Carboxygruppe darstellt, reduziert wird oder
c) zur Herstellung von Verbindungen der allgemeinen Formel I, in der $R_4$ eine Formylgruppe darstellt, eine Verbindung der allgemeinen Formel

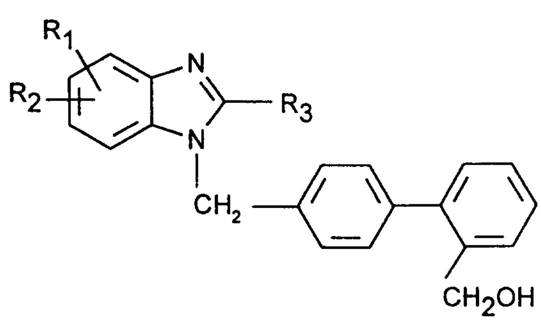

,( V )

in der
$R_1$ bis $R_3$ wie in den Ansprüchen 1 bis 6 definiert sind, oxydiert wird oder
d) zur Herstellung von Verbindungen der allgemeinen Formel I, in der $R_4$ eine Thiazolidin-2,4-dion-5-methylidengruppe darstellt, eine Verbindung der allgemeinen Formel

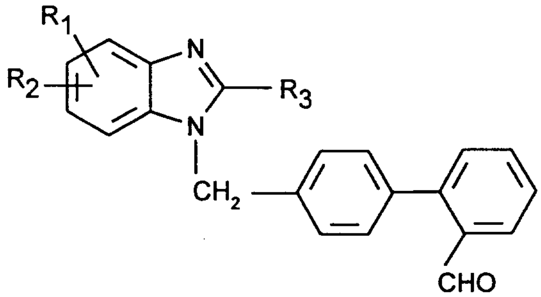

,( VI )

in der
$R_1$ bis $R_3$ wie in den Ansprüchen 1 bis 6 definiert sind, mit Thiazolidin-2,4-dion umgesetzt wird oder
e) zur Herstellung von Verbindungen der allgemeinen Formel I, in der $R_4$ eine gegebenenfalls durch eine Alkoxycarbonylgruppe mit insgesamt 2 bis 6 Kohlenstoffatomen substituierte Aminogruppe darstellt, eine gegebenenfalls im Reaktionsgemisch gebildete Verbindung der allgemeinen Formel

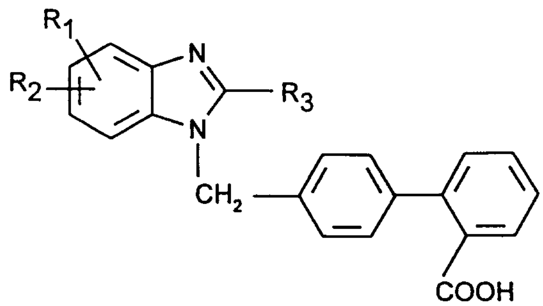

,( VII )

in der
$R_1$ bis $R_3$ wie in den Ansprüchen 1 bis 6 definiert sind, mit Natriumazid umgesetzt, anschließend mit Wasser oder einem aliphatischen Alkohol mit 1 bis 5 Kohlenstoffatomen umgesetzt und anschließend eine gegebenenfalls vorhandene Alkoxycarbonylgruppe mit 2 bis 6 Kohlenstoffatomen abgespalten wird oder
f) zur Herstellung von Verbindungen der allgemeinen Formel I, in der $R_4$ eine Sulfogruppe darstellt, eine Verbindung der allgemeinen Formel

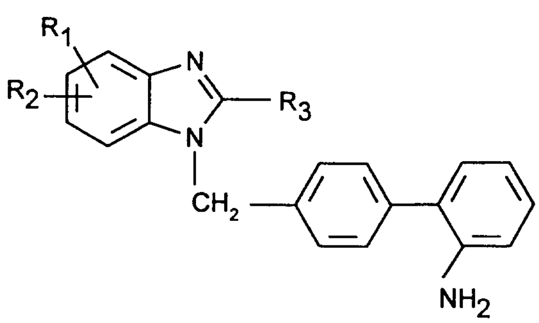

,( VIII )

in der

$R_1$ bis $R_3$ wie in den Ansprüchen 1 bis 6 definiert sind, mit einem Nitrit und anschließend mit Schwefeldioxid umgesetzt wird oder

g) zur Herstellung von Verbindungen der allgemeinen Formel I, in der $R_4$ eine durch eine Alkoxycarbonylgruppe mit insgesamt 2 bis 6 Kohlenstoffatomen, durch eine Trifluoracetyl- oder Trifluormethylsulfonylgruppesubstituierte Aminogruppe darstellt, eine Verbindung der allgemeinen Formel

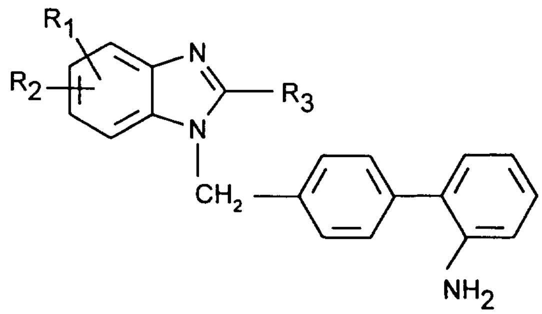

,( VIII )

in der

$R_1$ bis $R_3$ wie in den Ansprüchen 1 bis 6 definiert sind, mit einer Verbindung der allgemeinen Formel

$Z_2$-X-$R_c$ , (IX)

in der

$R_c$ eine Alkoxygruppe mit 1 bis 6 Kohlenstoffatomen oder eine Trifluormethylgruppe,

X eine Carbonyl- oder Sulfonylgruppe und

$Z_2$ eine Austrittsgruppe oder, falls $R_c$ eine Trifluormethylgruppe bedeutet, auch eine Hydroxygruppe bedeuten, umgesetzt wird oder

h) zur Herstellung von Verbindungen der allgemeinen Formel I, in der $R_4$ eine Sulfamoylgruppe darstellt, eine Verbindung der allgemeinen Formel

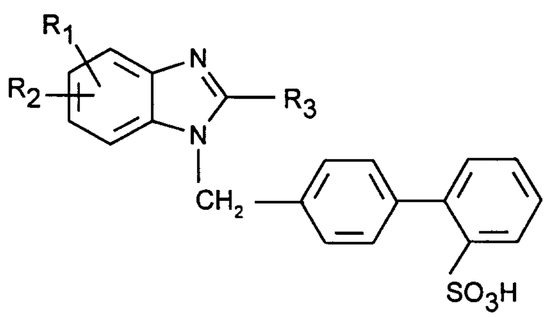

,( X )

in der

$R_1$ bis $R_3$ wie in den Ansprüchen 1 bis 6 definiert sind, oder deren reaktionsfähige Derivate mit Ammoniak umgesetzt wird oder

i) zur Herstellung einer Verbindung der allgemeinen Formel I, in der $R_4$ eine Sulfamoylgruppe darstellt, ein Schutzrest von einer Verbindung der allgemeinen Formel

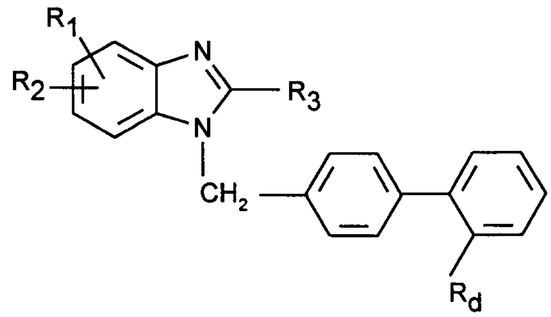

,( XI )

in der
$R_1$ bis $R_3$ wie in den Ansprüchen 1 bis 6 definiert sind und
$R_c$ eine mittels Hydrolyse, Aminolyse oder Behandlung mit Säuren in eine Sulfamoylgruppe überführbare Gruppe darstellt, abgespalten wird oder
j) zur Herstellung von Verbindungen der allgemeinen Formel I, in der $R_4$ eine durch eine Alkylcarbonylamino-, Alkoxycarbonylamino-, Alkylaminocarbonylamino-, Dialkylaminocarbonylamino-, Cycloalkylcarbonylamino-, Cycloalkylaminocarbonylamino-, Phenylcarbonylamino-, Phenylaminocarbonylamino-, Phenylalkylcarbonylamino- oder Phenylalkylaminocarbonylaminogruppe substituierte Sulfonylgruppe, in denen der Alkylteil jeweils 1 bis 3 Kohlenstoffatome, der Cycloalkylteil jeweils 5 bis 7 Kohlenstoffatome enthalten und der Phenylkern jeweils durch ein Fluor-, Chlor- oder Bromatom oder durch eine Methoxygruppe substituiert sein kann, darstellt, eine Verbindung der allgemeinen Formel

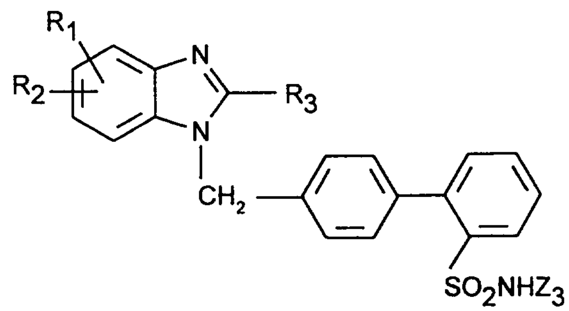

,( XII )

mit einer Verbindung der allgemeinen Formel

$Z_4$-$R_e$ , (XIII)

in denen
$R_1$ bis $R_3$ wie in den Ansprüchen 1 bis 6 definiert sind,
$Z_3$ eine Alkoxycarbonylgruppe mit insgesamt 2 bis 6 Kohlenstoffatomen,
$Z_4$ ein Wasserstoffatom und
$R_e$ eine Alkylamino-, Dialkylamino-, Cycloalkylamino-, Phenylamino- oder Phenylalkylaminogruppe oder
$Z_3$ ein Wasserstoffatom,
$Z_4$ eine $Z_5$-CO-Gruppe, in der $Z_5$ eine Austrittsgruppe oder auch $Z_5$ zusammen mit dem Wasserstoffatom einer zur Carbonylgruppe benachbarten Iminogruppe eine weitere Kohlenstoff-Stickstoff-Bindung darstellt, und
$R_e$ eine Alkyl-, Alkoxy-, Alkylamino-, Dialkylamino-, Cycloalkyl-, Cycloalkylamino-, Phenyl-, Phenylamino-, Phenylalkyl- oder Phenylalkylaminogruppe substituierte Sulfonylgruppe, wobei in den vorstehend erwähnten Gruppen der Alkylteil jeweils 1 bis 3 Kohlenstoffatome, der Cycloalkylteil jeweils 5 bis 7 Kohlenstoffatome enthalten und der Phenylkern jeweils durch ein Fluor-, Chlor- oder Bromatom oder durch eine Methoxygruppe substituiert sein kann, bedeuten, umgesetzt wird oder
k) zur Herstellung von Verbindungen der allgemeinen Formel I, in der $R_4$ eine 2,5-Dihydro-5-oxo-1,2,4-oxadiazol-3-yl-Gruppe darstellt, eine gegebenenfalls im Reaktionsgemisch erhaltene Verbin-

dung der allgemeinen Formel

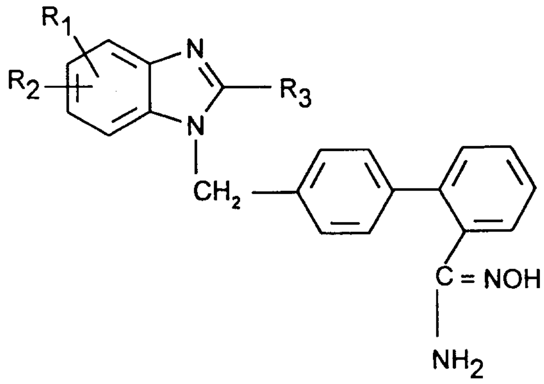

,( XIV )

in der
$R_1$ bis $R_3$ wie in den Ansprüchen 1 bis 6 definiert sind, mit einer Verbindung der allgemeinen Formel

$Z_6$-CO-$OR_f$ ,      (XV)

in der
$Z_6$ eine nukleophile Austrittsgruppe und
$R_f$ eine Alkyl-, Aryl- oder Aralkylgruppe bedeuten, umgesetzt und anschließend ein so erhaltenes acyliertes Amidoxim cyclisiert wird und
erforderlichenfalls ein während der Umsetzungen a) bis k) zum Schutze von reaktiven Gruppen verwendeter Schutzrest abgespalten wird und/oder
anschließend von einem so erhaltenen 1-, 3-Isomerengemich einer Verbindung der allgemeinen Formel I mittels Isomerentrennung das 1-Isomere abgetrennt wird oder
eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

Europäisches Patentamt

# EUROPÄISCHER RECHERCHENBERICHT

Nummer der Anmeldung
EP 95 10 3231

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP-A-0 566 020 (DR. KARL THOMAE GMBH) * Beispiel 66 * | 1,9 | C07D403/04 C07D413/14 C07D417/04 C07D417/14 C07D471/04 A61K31/415 C07D235/08 C07D235/18 |
| Y | EP-A-0 520 423 (TAKEDA CHEMICAL INDUSTRIES LTD.) * examples 1,4,30-38,52,54,57-59 * | 1,9 | |
| Y | EP-A-0 459 136 (TAKEDA CHEMICAL INDUSTRIES LTD.) * Anspruch 1 * | 1,9 | |
| Y | EP-A-0 400 835 (MERCK & CO., INC.) * R1=b,c,e,f,i,o; R8=c,z,bb,dd * | 1,9 | |
| A | EP-A-0 392 317 (DR. KARL THOMAE GMBH) * Seite 158, Zeile 8,9,49; Seite 159, Zeile 17-19 * | 1,9 | |
| Y | EP-A-0 556 789 (DR. KARL THOMAE GMBH) * Beispiele 5,6,9 * | 1,9 | RECHERCHIERTE SACHGEBIETE (Int.Cl.6) |
| Y | EP-A-0 543 263 (DR. KARL THOMAE GMBH) * Beispiele * | 1,9 | C07D A61K |
| Y,D | EP-A-0 502 314 (DR. KARL THOMAE GMBH) * R4=Seite 55, Zeile 25 * | 1,9 | |
| Y | DE-A-42 12 748 (DR. KARL THOMAE GMBH) * R3=Seite 3-8 * | 1,9 | |
| Y | EP-A-0 468 470 (DR. KARL THOMAE GMBH) * Beispiele * | 1,9 | |

Der vorliegende Recherchenbericht wurde für alle Patentansprüche erstellt

| Recherchenort | Abschlußdatum der Recherche | Prüfer |
|---|---|---|
| BERLIN | 21. August 1995 | Frelon, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)